(19) Europäisches Patentamt
European Patent Office
Office européen des brevets

(11) EP 2 751 266 B1

(12) **EUROPEAN PATENT SPECIFICATION**

(45) Date of publication and mention of the grant of the patent:
**29.03.2017 Bulletin 2017/13**

(21) Application number: **12759776.3**

(22) Date of filing: **21.09.2012**

(51) Int Cl.:
*C12N 9/52* (2006.01) *C11D 3/386* (2006.01)
*A23K 20/189* (2016.01)

(86) International application number:
**PCT/EP2012/068676**

(87) International publication number:
**WO 2013/041689 (28.03.2013 Gazette 2013/13)**

(54) **POLYPEPTIDES HAVING PROTEASE ACTIVITY AND POLYNUCLEOTIDES ENCODING SAME**

POLYPEPTIDE MIT PROTEASEAKTIVITÄT UND DIESE KODIERENDE POLYNUKLEOTIDE

POLYPEPTIDES DOTÉS D'UNE ACTIVITÉ DE PROTÉASE ET POLYNUCLÉOTIDES CODANT POUR CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.09.2011 EP 11182325**

(43) Date of publication of application:
**09.07.2014 Bulletin 2014/28**

(73) Proprietor: **Novozymes A/S**
**2880 Bagsvaerd (DK)**

(72) Inventors:
- **TAMS, Jeppe, Wegener**
  **DK-2820 Gentofte (DK)**
- **HOFF, Tine**
  **DK-2840 Holte (DK)**
- **GJERMANSEN, Morten**
  **DK-2670 Greve (DK)**
- **OESTERGAARD, Peter, Rahbek**
  **DK-2830 Virum (DK)**
- **OLINSKI, Robert, Piotr**
  **DK-3500 Vaerloese (DK)**
- **PONTOPPIDAN, Katrine**
  **DK-3540 Lynge (DE)**
- **SJOEHOLM, Carsten**
  **DK-2830 Virum (DK)**

(56) References cited:
**WO-A2-01/58276** **WO-A2-2004/072279**
**WO-A2-2005/052161**

- **DATABASE UniProt [Online] 2 March 2010 (2010-03-02), "SubName: Full=Peptidase alpha-lytic pro domain protein; Flags: Precursor;", XP002686536, retrieved from EBI accession no. UNIPROT:D2PRB9 Database accession no. D2PRB9**
- **DATABASE UniProt [Online] 17 April 2007 (2007-04-17), "SubName: Full=Serine protease;", XP002686537, retrieved from EBI accession no. UNIPROT:A4F726 Database accession no. A4F726**
- **PAGE M J ET AL: "Serine peptidases: Classification, structure and function", CMLS CELLULAR AND MOLECULAR LIFE SCIENCES, BIRKHÄUSER-VERLAG, BA, vol. 65, no. 7-8, 9 February 2008 (2008-02-09), pages 1220-1236, XP019619934, ISSN: 1420-9071**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### REFERENCE TO A SEQUENCE LISTING

This application contains a Sequence Listing in computer readable form.

### BACKGROUND OF THE INVENTION

### Field of the Invention

The present invention relates to isolated polypeptides having protease activity and isolated nucleic acid sequences encoding the proteases. The invention also relates to nucleic acid constructs, vectors, and host cells, including plant and animal cells, comprising the nucleic acid sequences, as well as methods for producing and using the proteases, in particular the use of the proteases in animal feed, and detergents.

### DESCRIPTION OF THE RELATED ART

The present invention provides polypeptides having protease activity and polynucleotides encoding the polypeptides.

The detergent industry has for more than 30 years implemented different enzymes in detergent formulations, most commonly used enzymes includes proteases, amylases and lipases each adapted for removing various types of stains. In addition to the enzymes detergent compositions typically include a complex combination of ingredients. For example, most cleaning products include surfactant system, bleaching agents or builders. Despite the complexity of current detergents, there remains a need for developing new detergent compositions comprising new enzymes and/or enzyme blends.

Traditionally laundering has been done at elevated temperatures and well known detergents have been selected to perform at higher temperatures.

The increased focus on improving the washing processes in order to make them more environmental friendly has resulted in a global tendency to lowering wash time, pH and temperature and decreasing the amount of detergent components which may influence the environment negatively.

There is therefore a desire to launder at lower temperature and therefore a need for detergent proteases having high performance at low temperatures.

In the use of proteases in animal feed (*in vivo*), and/or the use of such proteases for treating vegetable proteins (*in vitro)* it is noted that proteins are essential nutritional factors for animals and humans. Most livestock and many human beings get the necessary proteins from vegetable protein sources. Important vegetable protein sources are e.g. oilseed crops, legumes and cereals.

When e.g. soybean meal is included in the feed of mono-gastric animals such as pigs and poultry, a significant proportion of the soybean meal solids is not digested efficiently (the apparent ileal protein digestibility in piglets, growing pigs and poultry such as broilers, laying hens and roosters is only around 80%).

The gastrointestinal tract of animals consists of a series of segments each representing different pH environments. In mono-gastric animals such as pigs and poultry and many fish the stomach exhibits strongly acidic pH as low as pH 1-2, while the intestine exhibit a more neutral pH in the area pH 6-7. Poultry in addition to stomach and intestine also have a crop preceding the stomach, pH in the crop is mostly determined by the feed ingested and hence typically lies in the range pH 4-6. Protein digestion by a protease may occur along the entire digestive tract, given that the protease is active and survives the conditions in the digestive tract. Hence, proteases which are highly acid stable for survival in the gastric environment and at the same time are efficiently active at broad physiological pH of the target animal are especially desirable.

Also, animal feed is often formulated in pelleted form, where steam is applied in the pelleting process. It is therefore also desireable that proteases used in animal feed are capable to remain active after exposure to steam treatment

The use of proteases in animal feed to improve digestion of proteins in the feed is known. WO 95/28850 discloses the combination of a phytase and one or more microbial proteolytic enzymes to improve the solubility of vegetable proteins. WO 01/58275 discloses the use of acid stable proteases of the subtilisin family in animal feed. WO 01/58276 discloses the use in animal feed of acid-stable proteases related to the protease derived from Nocardiopsis sp. NRRL 18262 (the 10R protease), as well as a protease derived from Nocardiopsis alba DSM 14010. WO 04/072221, WO 04/111220, WO 04/111223, WO 05/035747, and WO 05/123911 disclose proteases related to the 10R protease and their use in animal feed. Also, WO 04/072279 discloses the use of other proteases.

WO 04/034776 discloses the use of a subtilisin/keratinase, PWD-1 from *B. licheniformis* in the feed of poultry.

WO 04/077960 discloses a method of increasing digestibility of forage or grain in ruminants by applying a bacterial or fungal protease.

Commercial products comprising a protease and marketed for use in animal feed include RONOZYME® ProAct (DSM NP/Novozymes), Axtra® (Danisco), Avizyme® (Danisco), Porzyme® (Danisco), Allzyme™ (Alltech), Versazyme® (BioResources, Int.), Poultrygrow™ (Jefo) and Cibenza® DP100 (Novus).

Database UniProt 2 March 2010 disclose peptidase alpha-lytic prodomain protein from Kribbella flavida, strain DSM17836/JCM10339/NBRC14399.

## SUMMARY OF THE INVENTION

The present invention relates to an isolated polypeptide having protease activity, selected from the group consisting of:

(a) a polypeptide having at least least 85%sequence identity to the mature polypeptide of SEQ ID NO: 2, wherein the mature polypeptide is amino acids 189 to 374 of SEQ ID NO: 2;
(b) a polypeptide encoded by a polynucleotide having at least 85%sequence identity to the mature polypeptide coding sequence of SEQ ID NO: 1, wherein the mature polynucleotide is nucleotides 665 to 1222 of SEQ ID NO: 1; and
(c) a fragment of the polypeptide of (a) or (b) that has protease activity, wherein the polypeptide has improved protease activity between 37°C and 60°C, compared to protease 10R (SEQ ID NO 7).

The present invention also relates to isolated polynucleotides encoding the polypeptides of the present invention; nucleic acid constructs; recombinant expression vectors; recombinant host cells comprising the polynucleotides; and methods of producing the polypeptides.

The present invention also relates to the use of the proteases of the invention in animal feed and in detergents, methods of producing animal feed compositions and detergent compositions, and animal feed compositions and detergent compositions

The present disclosure also relates to a polynucleotide encoding a signal peptide comprising or consisting of amino acids 1 to 29 of SEQ ID NO: 2, a polynucleotide encoding a propeptide comprising or consisting of amino acids 30 to 188 of SEQ ID NO: 2, or a polynucleotide encoding a signal peptide and a propeptide comprising or consisting of amino acids 1 to 188 of SEQ ID NO: 2, each of which is operably linked to a gene encoding a protein; nucleic acid constructs, expression vectors, and recombinant host cells comprising the polynucleotides; and methods of producing a protein.

## SHORT DESCRIPTION OF THE FIGURES

Figure 1 shows the activity on soybean-maize meal of the S1 protease from *Saccharothrix australiensis* compared to the 10R protease.

## Definitions

### Polypeptides Having Protease Activity

Polypeptides having protease activity, or proteases, are sometimes also designated peptidases, proteinases, peptide hydrolases, or proteolytic enzymes. Proteases may be of the exo-type that hydrolyse peptides starting at either end thereof, or of the endo-type that act internally in polypeptide chains (endopeptidases). Endopeptidases show activity on N- and C-terminally blocked peptide substrates that are relevant for the specificity of the protease in question.

The term "protease" is defined herein as an enzyme that hydrolyses peptide bonds. This definition of protease also applies to the protease-part of the terms "parent protease" and "protease variant," as used herein. The term "protease" includes any enzyme belonging to the EC 3.4 enzyme group (including each of the thirteen subclasses thereof). The EC number refers to Enzyme Nomenclature 1992 from NC-IUBMB, Academic Press, San Diego, California, including supplements 1-5 published in Eur. J. Bio-chem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively. The nomenclature is regularly supplemented and updated; see e.g. the World Wide Web (WWW) at http://www.chem.qmw.ac.uk/iubmb/enzyme/index.html.

The proteases of the invention and for use according to the invention are selected from the group consisting of:

(a) proteases belonging to the EC 3.4.21. enzyme group; and/or

(b) Serine proteases of the peptidase family S1;

as described in Biochem.J. 290:205-218 (1993) and in MEROPS protease database, release, 9.4 (www.merops.ac.uk). The database is described in Rawlings, N.D., Barrett, A.J. & Bateman, A. (2010) MEROPS: the peptidase database. Nucleic Acids Res 38, D227-D233.

For determining whether a given protease is a Serine protease, and a family S1 protease, reference is made to the above Handbook and the principles indicated therein. Such determination can be carried out for all types of proteases, be it naturally occurring or wild-type proteases; or genetically engineered or synthetic proteases.

The peptidases of family S1 contain the catalytic triad in the order His, Asp, Ser. Mutation of any of the amino acids of the catalytic triad will result in change or loss of enzyme activity. The amino acids of the catalytic triad of the S1 protease from *Saccharothrix australiensis* (SEQ ID NO: 2) are probably positions His-32, Asp-56 and Ser-136.

Protease activity can be measured using any assay, in which a substrate is employed, that includes peptide bonds relevant for the specificity of the protease in question. Assay-pH and assay-temperature are likewise to be adapted to the protease in question. Examples of assay-pH-values are pH 2, 3, 4, 5, 6, 7, 8, 9, 10, 11, or 12. Examples of assay-temperatures are 15, 20, 25, 30, 35, 37, 40, 45, 50, 55, 60, 65, 70, 80, 90, or 95°C. Examples of protease substrates are casein, such as Azurine-Crosslinked Casein (AZCL-casein), or suc-AAPF-pNA, Examples of suitable protease assays are described in the experimental part.

**Protease activity:** The term "protease activity" means a proteolytic activity (EC 3.4.21.) that catalyzes the hydrolysis of amide bond or a protein by hydrolysis of the peptide bond that link amino acids together in a polypeptide chain. Several assays for determining protease activity is available in the art. For purposes of the present invention, protease activity may be determined using Protazyme AK tablet (cross-linked and dyed casein; from Megazyme) as described in the Examples of the present application.The polypeptides of the present invention have at least 20%, e.g., at least 40%, at least 50%, at least 60%, at least 70%, at least 80%, at least 90%, at least 95%, or at least 100% of the protease activity of the mature polypeptide of SEQ ID NO: 2.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Cleaning compostions:** The term "cleaning compositions" and "cleaning formulations," refer to compositions that find use in the removal of undesired compounds from items to be cleaned, such as fabric, carpets, dishware including glassware, contact lenses, hard surfaces such as tiles, zincs, floors, and table surfaces, hair (shampoos), skin (soaps and creams), teeth (mouthwashes, toothpastes), etc. The terms encompasses any materials/compounds selected for the particular type of cleaning composition desired and the form of the product (e.g., liquid, gel, granule, or spray compositions), as long as the composition is compatible with the protease according to the invention and other enzyme(s) used in the composition. The specific selection of cleaning composition materials is readily made by considering the surface, item or fabric to be cleaned, and the desired form of the composition for the cleaning conditions during use. These terms further refer to any composition that is suited for cleaning, bleaching, disinfecting, and/or sterilizing any object and/or surface. It is intended that the terms include, but are not limited to detergent composition (e.g., liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish detergents).

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the

coding region of the polynucleotide encoding a polypeptide.

**Detergent compositon:** The term "detergent composition", includes unless otherwise indicated, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels, foam baths; metal cleaners; as well as cleaning auxiliaries such as bleach additives and "stain-stick" or pre-treat types. The terms "detergent composition" and "detergent formulation" are used in reference to mixtures which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the protease according to the invention, the term encompasses detergents that contains, e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti corrosion agents, enzyme inhibitors or stabilizers, enzyme activators, transferase(s), hydrolytic enzymes, oxido reductases, bluing agents and fluorescent dyes, antioxidants, and solubilizers.

**Dish washing composition:** The term "dish washing composition" refers to all forms of compositions for cleaning hard surfaces. The present invention is not restricted to any particular type of dish wash composition or any particular detergent.

**Enzyme Detergency benefit:** The term "enzyme detergency benefit" or "detergency" is defined herein as the advantageous effect an enzyme may add to a detergent compared to the same detergent without the enzyme. Important detergency benefits which can be provided by enzymes are stain removal with no or very little visible soils after washing and or cleaning, prevention or reduction of redeposition of soils released in the washing process an effect that also is termed anti-redeposition, restoring fully or partly the whiteness of textiles, which originally were white but after repeated use and wash have obtained a greyish or yellowish appearance an effect that also is termed whitening. Textile care benefits, which are not directly related to catalytic stain removal or prevention of redeposition of soils are also important for enzyme detergency benefits. Examples of such textile care benefits are prevention or reduction of dye transfer from one fabric to another fabric or another part of the same fabric an effect that is also termed dye transfer inhibition or anti-backstaining, removal of protruding or broken fibers from a fabric surface to decrease pilling tendencies or remove already existing pills or fuzz an effect that also is termed anti-pilling, improvement of the fabric-softness, colour clarification of the fabric and removal of particulate soils which are trapped in the fibers of the fabric or garment. Enzymatic bleaching is a further enzyme detergency benefit where the catalytic activity generally is used to catalyze the formation of bleaching component such as hydrogen peroxide or other peroxides.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fabric:** The term "fabric" encompasses any textile material. Thus, it is intended that the term encompass garments, as well as fabrics, yarns, fibers, non-woven materials, natural materials, synthetic materials, and any other textile material.

**Fragment:** The term "fragment" means a polypeptide having one or more (e.g., several) amino acids absent from the amino and/or carboxyl terminus of a mature polypeptide or domain; wherein the fragment has protease activity.

**Hard surface cleaning:** The term "Hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, and cutlery such as spoons, knives, forks, serving utensils, ceramics, plastics, metals, china, glass and acrylics.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Improved wash performance:** The term "improved wash performance" is defined herein as a (variant) enzyme (also a blend of enzymes, not necessarily only variants but also backbones, and in combination with certain cleaning composition etc.) displaying an alteration of the wash performance of a protease variant relative to the wash performance of the parent protease variant e.g. by increased stain removal. The term "wash performance" includes wash performance in laundry but also e.g. in dish wash.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance). An isolated substance may be present in a fermentation broth sample.

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 189 to 374 of SEQ ID NO: 2 based on amino acid sequencing using Edman degradation chemistry. It is known in the art that a host cell may produce a mixture of two of more different mature polypeptides (*i.e.*, with a different C-terminal and/or N-terminal amino acid) expressed by the same polynucleotide.

**Mature polypeptide coding sequence:** The term "mature polypeptide coding sequence" means a polynucleotide that encodes a mature polypeptide having protease activity. In one aspect, the mature polypeptide coding sequence is nucleotides 665 to 1222 of SEQ ID NO: 1.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and either 35% formamide, following standard Southern blotting procedures for 12 to 24 hours. The carrier material is finally washed three times each for 15 minutes using 2X SSC, 0.2% SDS at 60°C.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, *supra),* preferably version 5.0.0 or later. The parameters used are gap open penalty of 10, gap extension

penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (e.g., several) nucleotides absent from the 5' and/or 3' end of a mature polypeptide coding sequence; wherein the subsequence encodes a fragment having protease activity.

**Substantially pure polynucleotide:** The term "substantially pure polynucleotide" means a polynucleotide preparation free of other extraneous or unwanted nucleotides and in a form suitable for use within genetically engineered polypeptide production systems. Thus, a substantially pure polynucleotide contains at most 10%, at most 8%, at most 6%, at most 5%, at most 4%, at most 3%, at most 2%, at most 1 %, and at most 0.5% by weight of other polynucleotide material with which it is natively or recombinantly associated. A substantially pure polynucleotide may, however, include naturally occurring 5' and 3' untranslated regions, such as promoters and terminators. Preferably, the polynucleotide is at least 90% pure, *e.g.*, at least 92% pure, at least 94% pure, at least 95% pure, at least 96% pure, at least 97% pure, at least 98% pure, at least 99% pure, and at least 99.5% pure by weight. The polynucleotides of the present invention are preferably in a substantially pure form.

**Substantially pure polypeptide:** The term "substantially pure polypeptide" means a preparation that contains at most 10%, at most 8%, at most 6%, at most 5%, at most 4%, at most 3%, at most 2%, at most 1 %, and at most 0.5% by weight of other polypeptide material with which it is natively or recombinantly associated. Preferably, the polypeptide is at least 92% pure, e.g., at least 94% pure, at least 95% pure, at least 96% pure, at least 97% pure, at least 98% pure, at least 99%, at least 99.5% pure, and 100% pure by weight of the total polypeptide material present in the preparation. The polypeptides of the present invention are preferably in a substantially pure form. This can be accomplished, for example, by preparing the polypeptide by well known recombinant methods or by classical purification methods.

**Textile:** The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabbit and silk or synthetic polymer such as nylon, aramid, polyester, acrylic, polypropylene and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

**Textile care benefit:** "Textile care benefits", which are not directly related to catalytic stain removal or prevention of redeposition of soils, are also important for enzyme detergency benefits. Examples of such textile care benefits are prevention or reduction of dye transfer from one textile to another textile or another part of the same textile an effect that is also termed dye transfer inhibition or anti-backstaining, removal of protruding or broken fibers from a textile surface to decrease pilling tendencies or remove already existing pills or fuzz an effect that also is termed anti-pilling, improvement of the textile-softness, colour clarification of the textile and removal of particulate soils which are trapped in the fibers of the textile. Enzymatic bleaching is a further enzyme detergency benefit where the catalytic activity generally is used to catalyze the formation of bleaching component such as hydrogen peroxide or other peroxides or other bleaching species.

**Variant:** The term "variant" means a polypeptide having protease activity comprising an alteration, *i.e.*, a substitution, insertion, and/or deletion, at one or more (*e.g.*, several) positions. A substitution means replacement of the amino acid occupying a position with a different amino acid; a deletion means removal of the amino acid occupying a position; and an insertion means adding one or more (e.g several) amino acids, e.g. 1-5 amino acids adjacent to and immediately following the amino acid occupying a position. The variants of the present invention have at least 20%, *e.g.*, at least 40%, at least 50%, at least 60%, at least 70%, at least 80%, at least 90%, at least 95%, or at least 100% of the protease activity of the mature polypeptide of SEQ ID NO: 2.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Wash performance:** The term "wash performance" is used as an enzyme's ability to remove stains present on the object to be cleaned during e.g. wash or hard surface cleaning.

**Whiteness:** The term "Whiteness" is defined herein as a broad term with different meanings in different regions and for different customers. Loss of whiteness can e.g. be due to greying, yellowing, or removal of optical brighteners/hueing agents. Greying and yellowing can be due to soil redeposition, body soils, colouring from e.g. iron and copper ions or dye transfer. Whiteness might include one or several issues from the list below: Colorant or dye effects; Incomplete stain removal (e.g. body soils, sebum ect.); Re-deposition (greying, yellowing or other discolorations of the object) (removed soils re-associates with other part of textile, soiled or unsoiled); Chemical changes in textile during application; and Clarification or brightening of colours.

## DETAILED DESCRIPTION OF THE INVENTION

### Polypeptides Having protease Activity

In an embodiment, the present invention relates to an isolated polypeptide having a sequence identity to the mature polypeptide of SEQ ID NO: 2 of at least 85%which have protease activity, wherein the mature polypeptide is amino acids 189 to 374 of SEQ ID NO: 2, and wherein the polypeptide has improved protease activity between 37°C and 60°C, compared to protease 10R (SEQ ID NO 7).

. In one aspect, the polypeptide differ by no more than 20 amino acids, *e.g.*, 1, 2, 3, 4, 5, 6, 7, 8, 9, 10, 11, 12, 13, 14, 15, 16, 17, 18 or 19 from the mature polypeptide of SEQ ID NO: 2.

A polypeptide of the present invention preferably comprises or consists of the amino acid sequence of SEQ ID NO: 2; or is a fragment thereof having protease activity. In another aspect, the polypeptide comprises or consists of the mature polypeptide of SEQ ID NO: 2. In another aspect, the polypeptide comprises or consists of amino acids 189 to 374 of SEQ ID NO: 2.

In another embodiment, the present disclosure relates to an isolated polypeptide having protease activity encoded by a polynucleotide that hybridizes under very low stringency conditions, low stringency conditions, medium stringency conditions, medium-high stringency conditions, high stringency conditions, or very high stringency conditions with the mature polypeptide coding sequence of SEQ ID NO: 1, or the full-length complement thereof (Sambrook *et al.,* 1989, *Molecular Cloning, A Laboratory Manual,* 2d edition, Cold Spring Harbor, New York).

The polynucleotide of SEQ ID NO: 1 or a subsequence thereof, as well as the polypeptide of SEQ ID NO: 2 or a fragment thereof may be used to design nucleic acid probes to identify and clone DNA encoding polypeptides having protease activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, e.g., at least 25, at least 35, or at least 70 nucleotides in length. Preferably, the nucleic acid probe is at least 100 nucleotides in length, e.g., at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having protease activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or a subsequence thereof, the carrier material is used in a Southern blot.

For purposes of the present disclosure, hybridization indicates that the polynucleotide hybridizes to a labeled nucleic acid probe corresponding to (i) SEQ ID NO: 1; (ii) the mature polypeptide coding sequence of SEQ ID NO: 1; (iii) the full-length complement thereof; or (iv) a subsequence thereof; under very low to very high stringency conditions. Molecules to which the nucleic acid probe hybridizes under these conditions can be detected using, for example, X-ray film or any other detection means known in the art.

In one aspect, the nucleic acid probe is nucleotides 101 to 1405, nucleotides 188 to 1222, nucleotides 665 to 1222, or nucleotides 800 to 1200 of SEQ ID NO: 1. In another aspect, the nucleic acid probe is a polynucleotide that encodes the polypeptide of SEQ ID NO: 2; the mature polypeptide thereof; or a fragment thereof. In another aspect, the

nucleic acid probe is SEQ ID NO: 1.

In another embodiment, the present invention relates to an isolated polypeptide having protease activity encoded by a polynucleotide having a sequence identity to the mature polypeptide coding sequence of SEQ ID NO: 1 of at least 85%" wherein the mature polynucleotide is nucleotides 665 to 1222 of SEQ ID NO: 1, wherein the polypeptide has improved protease activity between 37°C and 60°C, compared to protease 10R (SEQ ID NO: 7).

In another embodiment, the present invention relates to variants of the mature polypeptide of SEQ ID NO: 2 comprising a substitution, deletion, and/or insertion at one or more (*e.g.,* several) positions. In an embodiment, the number of amino acid substitutions, deletions and/or insertions introduced into the mature polypeptide of SEQ ID NO: 2 is not more than 20, *e.g.,* 1, 2, 3, 4, 5, 6, 7, 8, 9, 10, 11, 12, 13, 14, 15, 16, 17, 18, or 19. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for protease activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide. In the polypeptide of the present invention essential amino acids forming the catalytic triad have been identified as amino acids corresponding to His-220, Asp-244 and Ser-324 in SEQ ID NO: 2 by alignment with the amino acid sequence of the 10R protease.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited

to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Embodiments**

In certain embodiments of the invention, the protease of the invention exhibits beneficial thermal properties such as thermostability, steam stability, pH properties, such as acid stability, etc.

An embodiment of the invention is isolated polypeptides having improved protease activity between 37°C and 60°C, such as between 37°C and 50°C, or at 37°C, 50°C or at 60°C compared to protease 10R.

Acidity/alkalinity properties

In certain embodiments of the invention the protease of the invention exhibits beneficial properties in respect of pH, such as acid stability etc. Stability of the protease at a low pH is beneficial since the protease can have activity in the intestine after passing through the stomach. Stability of the protease at a high pH is beneficial for cleaning and washing since detergent compositions often have an alkaline pH. In one embodiment of the invention, the protease retains >90% activity after 2 hours at pH 3 as determined using the method described in Example 4. In another embodiment of the invention, the protease retains >90% activity after 2 hours at pH 9 as determined using the method described in Example 4.

The present invention provides polypeptides having protease activity and polynucleotides encoding the polypeptides. The proteases of the invention are serine proteases of the peptidase family S1. The proteases of the invention exhibit surprising pH properties, especially pH stability properties which makes them interesting candidates for use in animal feed and/or detergents.

Wash Performance

In certain embodiments of the invention the protease of the invention exhibits beneficial wash performance. The S1 protease from *Saccharothrix australiensis* has is more effective at removing stains compared to detergent without any protease. The S1 protease from *Saccharothrix australiensis* is effective at removing blood and egg stains even at 20°C.

Feed application

The protease of the invention are active on Suc-Ala-Ala-Pro-Phe-pNA within a broad range from pH 4-11 and exhibit especially high activity in the range pH 6-11, are active on a feed relevant soybean meal-maize meal substrate within a broad physiological pH range from pH 3-7 and retains more than 80% activity after being subjected for 2 hours to pH as low as 2. Thus the S1 protease from *Saccharothrix australiensis* of the invention is suitable for various feed applications.

**Sources of Polypeptides Having protease Activity**

A polypeptide having protease activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a bacterial polypeptide. For example, the polypeptide may be a Gram-positive bacterial polypeptide such as a *Bacillus, Clostridium, Enterococcus, Geobacillus, Lactobacillus, Lactococcus, Oceanobacillus, Staphylococcus, Streptococcus, Saccharothrix* or *Streptomyces* polypeptide having protease activity, or a Gram-negative bacterial polypeptide such as a *Campylobacter, E. coli, Flavobacterium, Fusobacterium, Helicobacter, Ilyobacter, Neisseria, Pseudomonas, Salmonella,* or *Ureaplasma* polypeptide.

In one aspect, the polypeptide is a protease from a bacterium of the class *Actinobacteria,* such as from the order *Actinomycetales,* or from the suborder *Pseudonocardineae,* or from the family *Pseudonocardiaceae,* or from the genera *Saccharothrix.* In another aspect, the polypeptide is a *Saccharothrix australiensis* polypeptide.

Strains of these species are readily accessible to the public in a number of culture collections, such as the

American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (e.g., soil, composts, water, etc.) or DNA samples obtained directly from natural materials (e.g., soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.*, Sambrook *et al.,* 1989, *supra).*

**Polynucleotides**

The present invention also relates to isolated polynucleotides encoding a polypeptide of the present invention, as described herein.

The techniques used to isolate or clone a polynucleotide are known in the art and include isolation from genomic DNA or cDNA, or a combination thereof. The cloning of the polynucleotides from genomic DNA can be effected, *e.g.*, by using the well known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Saccharothrix,* or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, e.g., variants that differ in specific activity, thermostability, pH optimum, or the like. The variants may be constructed on the basis of the polynucleotide presented as the mature polypeptide coding sequence of SEQ ID NO: 1, *e.g.,* a subsequence thereof, and/or by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.*, Ford et al., 1991, Protein Expression and Purification 2: 95-107.

**Nucleic Acid Constructs**

The present invention also relates to nucleic acid constructs comprising a polynucleotide of the present invention operably linked to one or more control sequences that direct the expression of the coding sequence in a suitable host cell under conditions compatible with the control sequences.

A polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amy*Q), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention

in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease *(aprH), Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* anthranilate synthase, *Aspergillus nigerglucoamylase, Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs

the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease *(aprE), Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory systems are those that cause expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

## Expression Vectors

The present invention also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers

that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook *et al.,* 1989, *supra).*

**Host Cells**

The present invention also relates to recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus sub-*

*tilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, e.g., Chang and Cohen, 1979, Mol. Gen. Genet. 168: 111-115), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, *e.g.*, Shigekawa and Dower, 1988, Biotechniques 6: 742-751), or conjugation (see, *e.g.*, Koehler and Thorne, 1987, J. Bacteriol. 169: 5271-5278). The introduction of DNA into an *E. coli* cell may be effected by protoplast transformation (see, e.g., Hanahan, 1983, J. Mol. Biol. 166: 557-580) or electroporation (see, e.g., Dower et al., 1988, Nucleic Acids Res. 16: 6127-6145). The introduction of DNA into a *Streptomyces* cell may be effected by protoplast transformation, electroporation (see, e.g., Gong et al., 2004, Folia Microbiol. (Praha) 49: 399-405), conjugation (see, *e.g.*, Mazodier et al., 1989, J. Bacteriol. 171: 3583-3585), or transduction (see, *e.g.*, Burke et al., 2001, Proc. Natl. Acad. Sci. USA 98: 6289-6294). The introduction of DNA into a *Pseudomonas* cell may be effected by electroporation (see, *e.g.*, Choi et al., 2006, J. Microbiol. Methods 64: 391-397) or conjugation (see, *e.g.*, Pinedo and Smets, 2005, Appl. Environ. Microbiol. 71: 51-57). The introduction of DNA into a *Streptococcus* cell may be effected by natural competence (see, *e.g.*, Perry and Kuramitsu, 1981, Infect. Immun. 32: 1295-1297), protoplast transformation (see, *e.g.,* Catt and Jollick, 1991, Microbios 68: 189-207), electroporation (see, *e.g.*, Buckley et al., 1999, Appl. Environ. Microbiol. 65: 3800-3804), or conjugation (see, *e.g.*, Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and

*Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

**Methods of Production**

The present invention also relates to methods of producing a polypeptide of the present invention, comprising (a) cultivating a cell, which in its wild-type form produces the polypeptide, under conditions conducive for production of the polypeptide of the present invention; and (b) recovering the polypeptide of the present invention. In a preferred aspect, the cell is a *Saccharothrix* cell. In a more preferred aspect, the cell is a *Saccharothrix australiensis* cell.

The present invention also relates to methods of producing a polypeptide of the present invention, comprising (a) cultivating a recombinant host cell of the present invention under conditions conducive for production of the polypeptide of the present invention; and (b) recovering the polypeptide of the present invention.

The host cells are cultivated in a nutrient medium suitable for production of the polypeptide using methods known in the art. For example, the cell may be cultivated by shake flask cultivation, or small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation.

The polypeptide may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (e.g., ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Plants**

The present disclosure also relates to isolated plants, e.g., a transgenic plant, plant part, or plant cell, comprising a polynucleotide of the present disclosure so as to express and produce a polypeptide or domain in recoverable quantities. The polypeptide or domain may be recovered from the plant or plant part. Alternatively, the plant or plant part containing the polypeptide or domain may be used as such for improving the quality of a food or feed, *e.g.,* improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as Festuca, Lolium, temperate grass, such as Agrostis, and cereals, *e.g.*, wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, *e.g.*, epidermis, mesophyll, parenchyme, vascular tissues, meristems. Specific plant cell compartments, such as chloroplasts, apoplasts, mitochondria, vacuoles, peroxisomes and cytoplasm are also considered to be a plant part. Furthermore, any plant cell, whatever the tissue origin, is considered to be a plant part. Likewise, plant parts such as specific tissues and cells isolated to facilitate the utilization of the disclosure are also considered plant parts, e.g., embryos, endosperms, aleurone and seed coats.

Also included within the scope of the present disclosure are the progeny of such plants, plant parts, and plant cells.

The transgenic plant or plant cell expressing the polypeptide or domain may be constructed in accordance with methods known in the art. In short, the plant or plant cell is constructed by incorporating one or more expression constructs encoding the polypeptide or domain into the plant host genome or chloroplast genome and propagating the resulting modified plant or plant cell into a transgenic plant or plant cell.

The expression construct is conveniently a nucleic acid construct that comprises a polynucleotide encoding a polypeptide or domain operably linked with appropriate regulatory sequences required for expression of the polynucleotide in the plant or plant part of choice. Furthermore, the expression construct may comprise a selectable marker useful for identifying plant cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the polypeptide or domain is desired to be expressed. For instance, the expression of the gene encoding a polypeptide or domain may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV, the maize ubiquitin 1, or the rice actin 1 promoter may be used (Franck et al., 1980, Cell21: 285-294; Christensen et al., 1992, Plant Mol. Biol. 18: 675-689; Zhang et al., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoterfrom rice (Wu et al., 1998, Plant Cell Physiol. 39: 885-889), a *Vicia faba* promoter from the legumin B4 and the unknown seed protein gene from *Vicia faba* (Conrad et al., 1998, J. Plant Physiol. 152: 708-711), a promoter from a seed oil body protein (Chen et al., 1998, Plant Cell Physiol. 39: 935-941), the storage protein *napa* promoter from *Brassica napus,* or any other seed specific promoter known in the art, *e.g.,* as described in WO 91/14772. Furthermore, the promoter may be a leaf specific promoter such as the *rbcs* promoter from rice or tomato (Kyozuka et al., 1993, Plant Physiol. 102: 991-1000), the chlorella virus adenine methyltransferase gene promoter (Mitra and Higgins, 1994, Plant Mol. Biol. 26: 85-93), the *aldP* gene promoter from rice (Kagaya et al., 1995, Mol. Gen. Genet. 248: 668-674), or a wound inducible promoter such as the potato *pin2* promoter (Xu et al., 1993, Plant Mol. Biol. 22: 573-588). Likewise, the promoter may be induced by abiotic treatments such as temperature, drought, or alterations in salinity or induced by exogenously applied substances that activate the promoter, e.g., ethanol, oestrogens, plant hormones such as ethylene, abscisic acid, and gibberellic acid, and heavy metals.

A promoter enhancer element may also be used to achieve higher expression of a polypeptide or domain in the plant. For instance, the promoter enhancer element may be an intron that is placed between the promoter and the polynucleotide encoding a polypeptide or domain. For instance, Xu et *al.*, 1993, *supra,* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including *Agrobacterium*-mediated transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

*Agrobacterium tumefaciens*-mediated gene transfer is a method for generating transgenic dicots (for a review, see Hooykas and Schilperoort, 1992, Plant Mol. Biol. 19: 15-38) and for transforming monocots, although other transformation methods may be used for these plants. A method for generating transgenic monocots is particle bombardment (microscopic gold or tungsten particles coated with the transforming DNA) of embryonic calli or developing embryos (Christou, 1992, Plant J. 2: 275-281; Shimamoto, 1994, Curr. Opin. Biotechnol. 5: 158-162; Vasil et al., 1992, Bio/Technology 10: 667-674). An alternative method for transformation of monocots is based on protoplast transformation as described by Omirulleh et al., 1993, Plant Mol. Biol. 21: 415-428. Additional transformation methods include those described in U.S. Patent Nos. 6,395,966 and 7,151,204.

Following transformation, the transformants having incorporated the expression construct are selected and regenerated into whole plants according to methods well known in the art. Often the transformation procedure is designed for the selective elimination of selection genes either during regeneration or in the following generations by using, for example, co-transformation with two separate T-DNA constructs or site specific excision of the selection gene by a specific recombinase.

In addition to direct transformation of a particular plant genotype with a construct of the present disclosure, transgenic plants may be made by crossing a plant having the construct to a second plant lacking the construct. For

example, a construct encoding a polypeptide or domain can be introduced into a particular plant variety by crossing, without the need for ever directly transforming a plant of that given variety. Therefore, the present disclosureencompasses not only a plant directly regenerated from cells which have been transformed in accordance with the present disclosure, but also the progeny of such plants. As used herein, progeny may refer to the offspring of any generation of a parent plant prepared in accordance with the present disclosure. Such progeny may include a DNA construct prepared in accordance with the present disclosure. Crossing results in the introduction of a transgene into a plant line by cross pollinating a starting line with a donor plant line. Non-limiting examples of such steps are described in U.S. Patent No. 7,151,204.

Plants may be generated through a process of backcross conversion. For example, plants include plants referred to as a backcross converted genotype, line, inbred, or hybrid.

Genetic markers may be used to assist in the introgression of one or more transgenes of the disclosurefrom one genetic background into another. Marker assisted selection offers advantages relative to conventional breeding in that it can be used to avoid errors caused by phenotypic variations. Further, genetic markers may provide data regarding the relative degree of elite germplasm in the individual progeny of a particular cross. For example, when a plant with a desired trait which otherwise has a non-agronomically desirable genetic background is crossed to an elite parent, genetic markers may be used to select progeny which not only possess the trait of interest, but also have a relatively large proportion of the desired germplasm. In this way, the number of generations required to introgress one or more traits into a particular genetic background is minimized.

The present disclosure also relates to methods of producing a polypeptide or domain of the present invention comprising (a) cultivating a transgenic plant or a plant cell comprising a polynucleotide encoding the polypeptide or domain under conditions conducive for production of the polypeptide or domain; and (b) recovering the polypeptide or domain.

**Signal Peptide and Propeptide**

The present disclosure also relates to an isolated polynucleotide encoding a signal peptide comprising or consisting of amino acids 1 to 29 of SEQ ID NO: 2. The present disclosure also relates to an isolated polynucleotide encoding a propeptide comprising or consisting of amino acids 30 to 188 of SEQ ID NO: 2. The present disclosure also relates to an isolated polynucleotide encoding a signal peptide and a propeptide comprising or consisting of amino acids 1 to 188 of SEQ ID NO: 2. The polynucleotides may further comprise a gene encoding a protein, which is operably linked to the signal peptide and/or propeptide. The protein is preferably foreign to the signal peptide and/or propeptide. In one aspect, the polynucleotide encoding the signal peptide is nucleotides 101 to 187 of SEQ ID NO: 1. In another aspect, the polynucleotide encoding the propeptide is nucleotides 188 to 664 of SEQ ID NO: 1. In another aspect, the polynucleotide encoding the signal peptide and the propeptide is nucleotides 101 to 664 of SEQ ID NO: 1.

The present disclosure also relates to nucleic acid constructs, expression vectors and recombinant host cells comprising such polynucleotides.

**Detergent compositions**

In one embodiment, the invention is directed to detergent compositions comprising an enzyme of the present invention in combination with one or more additional cleaning composition components.Thus one embodiment, the present invention relates to a detergent composition comprising an isolated polypeptide having a sequence identity to the mature polypeptide of SEQ ID NO: 2 of at least 85%,which have protease activity, and wherein the mature polypeptide is amino acids 189 to 374 of SEQ ID NO: 2.

The choice of additional components is within the skill of the artisan and includes conventional ingredients, including the exemplary non-limiting components set forth below. The choice of components may include, for fabric care, the consideration of the type of fabric to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

**Enzyme of the present invention**

In one embodiment of the present invention, the polypeptide of the present invention may be added to a detergent composition in an amount corresponding to 0.001-200 mg of protein, such as 0.005-100 mg of protein, preferably 0.01-50 mg of protein, more preferably 0.05-20 mg of protein, even more preferably 0.1-10 mg of protein per liter of wash liquor.

The enzyme(s) of the detergent composition of the invention may be stabilized using conventional stabilizing

agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708 or the variants according to the invention may be stabilized using peptide aldehydes or ketones such as described in WO 2005/105826 and WO 2009/118375.

A polypeptide of the present invention may also be incorporated in the detergent formulations disclosed in WO97/07202.

**Surfactants**

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1 % to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 1 % to about 40% by weight of a cationic surfactant. Non-limiting examples of cationic surfactants include alklydimethylehanolamine quat (ADMEAQ), cetyltrimethylammonium bromide (CTAB), dimethyldistearylammonium chloride (DSDMAC), alkylbenzyldimethylammonium, alkyl quaternary ammonium compounds, alkoxylated quaternary ammonium (AQA), and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

When included therein the detergent will usually contain from about 1% to about 40% by weight of a semipolar surfactant. Non-limiting examples of semipolar surfactants include amine oxides (AO) such as alkyldimethylamineoxide, *N*-(coco alkyl)-*N,N*-dimethylamine oxide and *N*-(tallow-alkyl)-*N,N*-bis(2-hydroxyethyl)amine oxide, fatty acid alkanolamides and ethoxylated fatty acid alkanolamides, and combinations thereof.

When included therein the detergent will usually contain from about 1 % to about 40% by weight of a zwitterionic surfactant. Non-limiting examples of zwitterionic surfactants include betaine, alkyldimethylbetaine, and sulfobetaine, and combinations thereof.

**Hydrotropes**

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see e.g. review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases.

However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

**Builders and Co-Builders**

The detergent composition may contain about 0-65% by weight, such as about 5% to about 50% of a detergent builder or co-builder, or a mixture thereof. In a dish wash deteregent, the level of builder is typically 40-65%, particularly 50-65%. The builder and/or co-builder may particularly be a chelating agent that forms water-soluble complexes with Ca and Mg. Any builder and/or co-builder known in the art for use in laundry detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI)" and combinations thereof.

The detergent composition may also contain 0-65% by weight, such as about 5% to about 40%, of a detergent co-builder, or a mixture thereof. The detergent composition may include include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-*N,N*-diacetic acid (GLDA), ethylenediaminetetra(methylenephosphonic acid) (HEDP), ethylenediaminetetra(methylenephosphonic acid) (EDTMPA), diethylenetriaminepenta(methylenephosphonic acid) (DTPMPA), *N*-(2-hydroxyethyl)iminodiacetic acid (EDG), aspartic acid-*N*-monoacetic acid (ASMA), aspartic acid-*N,N*-diacetic acid (ASDA), aspartic acid-*N*-monopropionic acid (ASMP), iminodisuccinic acid (IDA), N-(2-sulfomethyl)aspartic acid (SMAS), *N*-(2-sulfoethyl)aspartic acid (SEAS), *N*-(2-sulfomethyl)glutamic acid (SMGL), *N*-(2-sulfoethyl)glutamic acid (SEGL), *N*-methyliminodiacetic acid (MIDA), α-alanine-*N,N*-diacetic acid (α-ALDA), serine-*N,N*-diacetic acid (SEDA), isoserine-N,N-diacetic acid (ISDA), phenylalanine-*N,N*-diacetic acid (PHDA), anthranilic acid- *N,N*-diacetic acid (ANDA), sulfanilic acid-*N,N*-diacetic acid (SLDA), taurine-*N,N*-diacetic acid (TUDA) and sulfomethyl-*N,N*-diacetic acid (SMDA), *N*-(2-hydroxyethyl)-ethylidenediamine-*N*, *N'*, *N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053.

**Bleaching Systems**

The detergent may contain 0-10% by weight, such as about 1% to about 5%, of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides, Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)benzenesulfonate (NOBS), and/or those disclosed in

WO98/17767. A particular family of bleach activators of interest was disclosed in EP624154 and particulary preferred in that family is acetyl triethyl citrate (ATC). ATC or a short chain triglyceride like Triacin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i)
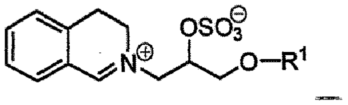

(ii)
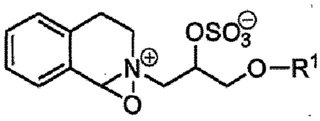

(iii) and mixtures thereof; wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, n- dodecyl, n-tetradecyl, n-hexadecyl, n-octadecyl, iso-nonyl, iso-decyl, iso- tridecyl and iso-pentadecyl. Other exemplary bleaching systems are described, e.g., in WO2007/087258, WO2007/087244, WO2007/087259, WO2007/087242. Suitable photobleaches may for example be sulfonated zinc phthalocyanine

**Polymers**

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

**Fabric hueing agents**

The detergent compositions of the present invention may also include fabric hueing agents such as dyes or pigments which when formulated in detergent compositions can deposit onto a fabric when said fabric is contacted with a wash liquor comprising said detergent compositions thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and r EP1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred

when the composition is in the form of a unit dose pouch. Suitable hueing agents are also disclosed in, e.g., WO 2007/087257, WO2007/087243.

**(Additional) Enzymes**

The detergent additive as well as the detergent composition may comprise one or more [additional] enzymes such as a protease, lipase, cutinase, an amylase, carbohydrase, cellulase, pectinase, mannanase, arabinase, galactanase, xylanase, oxidase, e.g., a laccase, and/or peroxidase.

In general the properties of the selected enzyme(s) should be compatible with the selected detergent, (*i.e.*, pH-optimum, compatibility with other enzymatic and non-enzymatic ingredients, etc.), and the enzyme(s) should be present in effective amounts.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (Genencor International Inc.), and KAC-500(B)™ (Kao Corporation).

Proteases: Suitable proteases include those of animal, vegetable or microbial origin. Microbial origin is preferred. Chemically modified or protein engineered mutants are included. The protease may be a serine protease or a metalloprotease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g., of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235, and 274.

Preferred commercially available protease enzymes include Alcalase™, Savinase™, Primase™, Duralase™, Esperase™, and Kannase™ (Novozymes A/S), Maxatase™, MaxacalTM, Maxapem™, Properase™, Purafect™, Purafect OxP™, FN2™, and FN3™ (Genencor International Inc.).

Lipases and Cutinases: Suitable lipases and cutinases include those of bacterial or fungal origin. Chemically modified or protein engineered mutants are included. Examples include lipase from *Thermomyces, e.g.,* from *T. lanuginosus* (previously named *Humicola lanuginosa*) as described in EP 258 068 and EP 305 216, cutinase from *Humicola, e.g. H. insolens* as described in WO 96/13580, a *Pseudomonas* lipase, *e.g.,* from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, *e.g.*, from *B. subtilis* (Dartois et al., 1993, Biochemica et Biophysica Acta, 1131: 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO2007/087508 and WO 2009/109500.

Preferred commercially available lipase enzymes include Lipolase™, Lipolase Ultra™, and Lipex™; Lecitase™, Lipolex™; Lipoclean™, Lipoprime™ (Novozymes A/S). Other commercially available lipases include Lumafast (Genencor Int Inc); Lipomax (Gist-Brocades/Genencor Int Inc) and Bacillus sp lipase from Solvay.

Amylases: Suitable amylases ($\alpha$ and/or $\beta$) include those of bacterial or fungal origin. Chemically modified or protein engineered mutants are included. Amylases include, for example, $\alpha$-amylases obtained from *Bacillus, e.g.,* a special strain of *Bacillus licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Stainzyme™, Natalase™, Duramyl™, Termamyl™, Fungamyl™ and BAN™ (Novozymes A/S), Rapidase™ and Purastar™ (from Genencor International Inc.).

Peroxidases/Oxidases: Suitable peroxidases/oxidases include those of plant, bacterial or fungal origin. Chemically modified or protein engineered mutants are included. Examples of useful peroxidases include peroxidases from

*Coprinus, e.g.*, from *C. cinereus,* and variants thereof as those described in WO 93/24618, WO 95/10602, and WO 98/15257.

Commercially available peroxidases include Guardzyme™ (Novozymes A/S).

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

**Adjunct materials**

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents - The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain additional components that may tint articles being cleaned, such as fluorescent whitening agent or optical brighteners. Where present the brightener is preferably at a level of about 0.01% to about 0.5%.. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate; 4,4'-bis-(2,4-dianilino-s-triazin-6-ylamino) stilbene-2.2'-disulfonate; 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-2,1,3-triazol-2-yl)stilbene-2,2'-disulfonate; 4,4'-bis-(2-anilino-4(1-methyl-2-hydroxy-ethylamino)-s-triazin-6-ylamino) stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

Soil release polymers - The detergent compositions of the present invention may also include one or more soil

release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof.

Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, structurants for liquid detergents and/or structure elasticizing agents.

**Formulation of detergent products**

The detergent composition of the invention may be in any convenient form, e.g., a bar, a homogenous tablet, a tablet having two or more layers, a regular or compact powder, a granule, a paste, a gel, or a regular, compact or concentrated liquid.

Detergent formulation forms: Layers (same or different phases), Pouches, versus forms for Machine dosing unit.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids. Ref: (US2009/0011970 A1)

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

Definition/characteristics of the forms:

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

### Laundry soap bars

The enzymes of the invention may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g a two stage vacuum plodder, extruders, cutters, logo-stampers, cooling tunnels and wrappers. The invention is not limited to preparing the laundry soap bars by any single method. The premix of the invention may be added to the soap at different stages of the process. For example, the premix containing a soap, an enzyme, optionally one or more additional enzymes, a protease inhibitor, and a salt of a monovalent cation and an organic anion may be prepared and and the mixture is then plodded. The enzyme and optional additional enzymes may be added at the same time as the protease inhibitor for example in liquid form. Besides the mixing step and the plodding step, the process may further comprise the steps of milling, extruding, cutting, stamping, cooling and/or wrapping.

### Granular detergent formulations

A granular detergent may be formulated as described in WO09/092699, EP1705241, EP1382668, WO07/001262, US6472364, WO04/074419 or WO09/102854. Other useful detergent formulations are described in WO09/124162, WO09/124163, WO09/117340, WO09/117341, WO09/117342, WO09/072069, WO09/063355, WO09/132870, WO09/121757, WO09/112296, WO09/112298, WO09/103822, WO09/087033, WO09/050026, WO09/047125, WO09/047126, WO09/047127, WO09/047128, WO09/021784, WO09/010375, WO09/000605, WO09/122125, WO09/095645, WO09/040544, WO09/040545, WO09/024780, WO09/004295, WO09/004294, WO09/121725, WO09/115391, WO09/115392, WO09/074398, WO09/074403, WO09/068501, WO09/065770, WO09/021813, WO09/030632, and WO09/015951.

WO2011025615, WO2011016958, WO2011005803, WO2011005623, WO2011005730, WO2011005844, WO2011005904, WO2011005630, WO2011005830, WO2011005912, WO2011005905, WO2011005910, WO2011005813, WO2010135238, WO2010120863, WO2010108002, WO2010111365, WO2010108000, WO2010107635, WO2010090915, WO2010033976, WO2010033746, WO2010033747, WO2010033897, WO2010033979, WO2010030540, WO2010030541, WO2010030539, WO2010024467, WO2010024469, WO2010024470, WO2010025161, WO2010014395, WO2010044905, WO2010145887, WO2010142503, WO2010122051, WO2010102861, WO2010099997, WO2010084039, WO2010076292, WO2010069742, WO2010069718, WO2010069957, WO2010057784, WO2010054986, WO2010018043, WO2010003783, WO2010003792, WO2011023716, WO2010142539, WO2010118959, WO2010115813, WO2010105942, WO2010105961, WO2010105962, WO2010094356, WO2010084203, WO2010078979, WO2010072456, WO2010069905, WO2010076165, WO2010072603, WO2010066486, WO2010066631, WO2010066632, WO2010063689, WO2010060821, WO2010049187, WO2010031607, WO2010000636,

**Uses**

The present invention is also directed to methods for using the protease according to the invention or compositions thereof in laundry of textilea and fabrics, such as house hold laundry washing and industrial laundry washing as well as for animal feed.

The invention is also directed to methods for using the compositions thereof in hard surface cleaning such as automated Dish Washing (ADW), car wash and cleaning of Industrial surfaces. Thus one embodiment, the present invention relates to the use in cleaning such as laundry or desh wash of a protease according to the invention or a detergent composition comprising a protease according to the present invention having a sequence identity to the mature polypeptide of Thus one embodiment, the present invention relates to the use of an isolated polypeptide having a sequence identity to the mature polypeptide of SEQ ID NO: 2 of at least 85%, which have protease activity in a detergent, a cleaning process and/or laundry process, wherein the mature polypeptide is amino acids 189 to 374 of SEQ ID NO: 2.

Use in detergents. The polypeptides of the present invention may be added to and thus become a component of a detergent composition.

The detergent composition of the present invention may be formulated, for example, as a hand or machine laundry detergent composition including a laundry additive composition suitable for pretreatment of stained fabrics and a rinse added fabric softener composition, or be formulated as a detergent composition for use in general household hard surface cleaning operations, or be formulated for hand or machine dishwashing operations.

In a specific aspect, the present invention provides a detergent additive comprising a polypeptide of the present invention as described herein.

**Use of proteases of the invention in Detergents**

The soils and stains that are important for detergent formulators are composed of many different substances, and a range of different enzymes, all with different substrate specificities have been developed for use in detergents both in relation to laundry and hard surface cleaning, such as dishwashing. These enzymes are considered to provide an enzyme detergency benefit, since they specifically improve stain removal in the cleaning process they are applied in as compared to the same process without enzymes. Stain removing enzymes that are known in the art include enzymes such as carbohydrases, amylases, proteases, lipases, cellulases, hemicellulases, xylanases, cutinases, and pectinase.

In one aspect, the present invention concerns the use of an isolated polypeptide having a sequence identity to the mature polypeptide of SEQ ID NO: 2 of at least at least 85%, in detergent compositions and cleaning processes, such as laundry and hard surface cleaning, wherein the mature polypeptide is amino acids 189 to 374 of SEQ ID NO: 2. Thus, in one aspect, the present invention demonstrates the detergency effect of a variety of exemplary proteases of the invention on various stains and under various conditions. In a particular aspect of the invention the detergent composition and the use in cleaning process concerns the use of a protease of the invention together with at least one of the above mentioned stain removal enzymes.

In a preferred aspect of the present invention the protease of the invention useful according to the invention may be combined with at least two enzymes. These additional enzymes are described in details in the section "other enzymes", more preferred at least three, four or five enzymes. Preferably, the enzymes have different substrate specificity, e.g., carbolytic activity, proteolytic activity, amylolytic activity, lipolytic activity, hemicellulytic activity or pectolytic activity. The enzyme combination may for example be a protease of the invention with another stain removing enzyme, e.g., a protease of the inveniton and an amylase, a protease of the invention and a cellulase, a protease of the invention and a hemicellulase, a protease of the invention and a lipase, a protease of the invention and a cutinase, a protease of the invention and a pectinase or a protease of the invention and an anti-redeposition enzyme. More preferably, the protease of the invention is combined with at least two other stain removing enzymes, e.g., a protease of the invention, a lipase and an amylase; or a protease of the invention, an amylase and a pectinase; or a protease of the invention, an amylase and a cutinase; or a protease of the invention, an amylase and a cellulase; or a protease of the invention, an amylase and a hemicellulase; or a protease of the invention, a lipase and a pectinase; or a protease of the invention, a lipase and a cutinase; or a protease of the invention, a lipase and a cellulase; or a protease of the invention, a lipase and a hemicellulase. Even more preferably, a protease of the invention may be combined with at least three other stain removing enzymes, e.g., a protease of the invention, an amylase, a lipase and a pectinase; or a protease of the invention, an amylase, a lipase and a cutinase; or a protease of the invention, an amylase, a lipase and a cellulase; or a protease of the invention, an amylase, a lipase and a hemicellulase. A protease of the invention may be combined with any of the enzymes selected from the non-exhaustive list comprising: carbohydrases, such as an amylase, a hemicellulase, a pectinase, a cellulase, a xanthanase or a pullulanase, a peptidase, other proteases or a lipase.

In another embodiment of the present invention, a protease of the invention may be combined with one or more metalloproteases, such as a M4 Metalloprotease, including Neutrase™ or Thermolysin. Such combinations may further comprise combinations of the other detergent enzymes as outlined above.

The cleaning process or the textile care process may for example be a laundry process, a dishwashing process or cleaning of hard surfaces such as bathroom tiles, floors, table tops, drains, sinks and washbasins. Laundry processes can for example be household laundering, but it may also be industrial laundering. Furthermore, the invention relates to a process for laundering of fabrics and/or garments where the process comprises treating fabrics with a washing solution containing a detergent composition, and at least one protease of the invention. The cleaning process or a textile care process can for example be carried out in a machine washing process or in a manual washing process. The washing solution can for example be an aqueous washing solution containing a detergent composition.

The fabrics and/or garments subjected to a washing, cleaning or textile care process of the present invention may be conventional washable laundry, for example household laundry. Preferably, the major part of the laundry is garments and fabrics, including knits, woven, denims, non-woven, felts, yarns, and towelling. The fabrics may be cellulose based such as natural cellulosics, including cotton, flax, linen, jute, ramie, sisal or coir or manmade cellulosics (e.g., originating from wood pulp) including viscose/rayon, ramie, cellulose acetate fibers (tricell), lyocell or blends thereof. The fabrics may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g., polyamide fibers, acrylic fibers, polyester fibers, polyvinyl alcohol fibers, polyvinyl chloride fibers, polyurethane fibers, polyurea fibers, aramid fibers), and cellulose-containing fibers (e.g., rayon/viscose, ramie, flax, linen, jute, cellulose acetate fibers, lyocell).

The last few years there has been an increasing interest in replacing components in detergents, which is derived from petrochemicals with renewable biological components such as enzymes and polypeptides without compromising the wash performance. When the components of detergent compositions change new enzyme activities or new enzymes having alternative and/or improved properties compared to the common used detergent enzymes such as proteases, lipases and amylases is needed to achieve a similar or improved wash performance when compared to the traditional detergent compositions.

The invention further concerns the use of proteases of the invention a proteinaceous stain removing processes. The proteinaceous stains may be stains such as food stains, e.g., baby food, sebum, cocoa, egg, blood, milk, ink, grass, or a combination hereof.

Typical detergent compositions includes various components in addition to the enzymes, these components have different effects, some components like the surfactants lower the surface tension in the detergent, which allows the stain being cleaned to be lifted and dispersed and then washed away, other components like bleach systems removes discolor often by oxidation and many bleaches also have strong bactericidal properties, and are used for disinfecting and sterilizing. Yet other components like builder and chelator softens, e.g., the wash water by removing the metal ions form the liquid.

In a particular embodiment, the invention concerns the use of a composition comprising a protease of the invention, wherein said enzyme composition further comprises at least one or more of the following a surfactant, a builder, a chelator or chelating agent, bleach system or bleach component in laundry or dish wash.

In a preferred embodiment of the invention the amount of a surfactant, a builder, a chelator or chelating agent, bleach system and/or bleach component are reduced compared to amount of surfactant, builder, chelator or chelating agent, bleach system and/or bleach component used without the added protease of the invention. Preferably the at least one component which is a surfactant, a builder, a chelator or chelating agent, bleach system and/or bleach component is present in an amount that is 1% less, such as 2% less, such as 3% less, such as 4% less, such as 5% less, such as 6% less, such as 7% less, such as 8% less, such as 9% less, such as 10% less, such as 15% less, such as 20% less, such as 25% less, such as 30% less, such as 35% less, such as 40% less, such as 45% less, such as 50% less than the amount of the component in the system without the addition of protease of the invention, such as a conventional amount of such component. In one aspect, the protease of the invention is used in detergent compositions wherein said composition is free of at least one component which is a surfactant, a builder, a chelator or chelating agent, bleach system or bleach component and/or polymer.

**Washing Method**

The detergent compositions of the present invention are ideally suited for use in laundry applications. Accordingly, the present invention includes a method for laundering a fabric. The method comprises the steps of contacting a fabric to be laundered with a cleaning laundry solution comprising the detergent composition according to the invention. The fabric may comprise any fabric capable of being laundered in normal consumer use conditions. The solution preferably has a pH of from about 5.5 to about 9. The compositions may be employed at concentrations of from about 100 ppm, preferably 500 ppm to about 15,000 ppm in solution. The water temperatures typically range from about 5°C to about 90°C, including about 10°C, about 15°C, about 20°C, about 25°C, about 30°C, about 35°C, about 40°C, about 45°C, about 50°C, about 55°C, about 60°C, about 65°C, about 70°C, about 75°C, about 80°C, about 85°C and about 90°C.

The water to fabric ratio is typically from about 1:1 to about 30:1.

In particular embodiments, the washing method is conducted at a pH of from about 5.0 to about 11.5, or in alternative embodiments, even from about 6 to about 10.5, such as about 5 to about 11, about 5 to about 10, about 5 to about 9, about 5 to about 8, about 5 to about 7, about 5.5 to about 11, about 5.5 to about 10, about 5.5 to about 9, about 5.5 to about 8, about 5.5. to about 7, about 6 to about 11, about 6 to about 10, about 6 to about 9, about 6 to to about 8, about 6 to about 7, about 6.5 to about 11, about 6.5 to about 10, about 6.5 to about 9, about 6.5 to about 8, about 6.5 to about 7, about 7 to about 11, about 7 to about 10, about 7 to about 9, or about 7 to about 8, preferably about 5.5 to about 9, and more preferably about 6 to about 8.

In particular embodiments, the washing method is conducted at a degree of hardness of from about 0°dH to about 30°dH, such as about 1 °dH, about 2°dH, about 3°dH, about 4°dH, about 5°dH, about 6°dH, about 7°dH, about 8°dH, about 9°dH, about 10°dH, about 11°dH, about 12°dH, about 13°dH, about 14°dH, about 15°dH, about 16°dH, about 17°dH, about 18°dH, about 19°dH, about 20°dH, about 21°dH, about 22°dH, about 23°dH, about 24°dH, about 25°dH, about 26°dH, about 27°dH, about 28°dH, about 29°dH,about 30°dH. Under typical European wash conditions, the degree of hardness is about 15°dH, under typical US wash conditions about 6°dH, and under typical Asian wash conditions, about 3°dH.

The present invention relates to a method of cleaning a fabric, a dishware or hard surface with a detergent composition comprising a protease of the invention.

A preferred embodiment concerns a method of cleaning, said method comprising the steps of: contacting an object with a cleaning composition comprising a protease of the invention under conditions suitable for cleaning said object. In a preferred embodiment the cleaning composition is a detergent composition and the process is a laundry or a dish wash process.

Still another embodiment relates to a method for removing stains from fabric which comprises contacting said a fabric with a composition comprising a protease of the invention under conditions suitable for cleaning said object.

In a preferred embodiment the compositions for use in the methods above further comprises at least one additional enzyme as set forth in the "other enzymes" section above, such as an enzyme selected from the group consisting of carbohydrases, peptidases, proteases, lipases, cellulase, xylanases or cutinases or a combination hereof. In yet another preferred embodiment the compositions comprises a reduced amount of at least one or more of the following components a surfactant, a builder, a chelator or chelating agent, bleach system or bleach component or a polymer.

Also contemplated are compositions and methods of treating fabrics (e.g., to desize a textile) using one or more of the protease of the invention. The protease can be used in any fabric-treating method which is well known in the art (see, e.g., U.S. Patent No. 6,077,316). For example, in one aspect, the feel and appearance of a fabric is improved by a method comprising contacting the fabric with a protease in a solution. In one aspect, the fabric is treated with the solution under pressure.

In one embodiment, the protease is applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The protease can be applied to remove these sizing protein or protein derivatives. After the textiles have been woven, a fabric can proceed to a desizing stage. This can be followed by one or more additional fabric processing steps. Desizing is the act of removing size from textiles. After weaving, the size coating should be removed before further processing the fabric in order to ensure a homogeneous and wash- proof result. Also provided is a method of desizing comprising enzymatic hydrolysis of the size by the action of an enzyme.

## Low Temperature Uses

One embodiment of the invention concerns a method of doing laundry, dish wash or industrial cleaning comprising contacting a surface to be cleaned with a protease of the invention, and wherein said laundry, dish wash, industrial or institutional cleaning is performed at a temperature of about 40°C or below. One embodiment of the invention relates to the use of a protease of the invention in laundry, dish wash or a cleaning process wherein the temperature in laundry, dish wash, industrial cleaning is about 40°C or below.

In another embodiment, the invention concerns the use of a protease of the invention in a protein removing process, wherein the temperature in the protein removing process is about 40°C or below.

The present disclosure also relates to the use in laundry, dish wash or industrial cleaning process of a protease of the disclosure having at least one improved property compared to Savinase (SEQ ID NO 6) and wherein the temperature in laundry, dish wash or cleaning process is performed at a temperature of about 40°C or below.

In each of the above-identified methods and uses, the wash temperature is about 40°C or below, such as about 39°C or below, such as about 38°C or below, such as about 37°C or below, such as about 36°C or below, such as about

35°C or below, such as about 34°C or below, such as about 33°C or below, such as about 32°C or below, such as about 31°C or below, such as about 30°C or below, such as about 29°C or below, such as about 28°C or below, such as about 27°C or below, such as about 26°C or below, such as about 25°C or below, such as about 24°C or below, such as about 23°C or below, such as about 22°C or below, such as about 21°C or below, such as about 20°C or below, such as about 19°C or below, such as about 18°C or below, such as about 17°C or below, such as about 16°C or below, such as about 15°C or below, such as about 14°C or below, such as about 13°C or below, such as about 12°C or below, such as about 11°C or below, such as about 10°C or below, such as about 9°C or below, such as about 8°C or below, such as about 7°C or below, such as about 6°C or below, such as about 5°C or below, such as about 4°C or below, such as about 3°C or below, such as about 2°C or below, such as about 1°C or below.

In another preferred embodiment, the wash temperature is in the range of about 5-40°C, such as about 5-30°C, about 5-20°C, about 5-10°C, about 10-40°C, about 10-30°C, about 10-20°C, about 15-40°C, about 15-30°C, about 15-20°C, about 20-40°C, about 20-30°C, about 25-40°C, about 25-30°C, or about 30-40°C. In a particular preferred embodiment the wash temperature is about 30°C.

In particular embodiments, the low temperature washing method is conducted at a pH of from about 5.0 to about 11.5, or in alternative embodiments, even from about 6 to about 10.5, such as about 5 to about 11, about 5 to about 10, about 5 to about 9, about 5 to about 8, about 5 to about 7, about 5.5 to about 11, about 5.5 to about 10, about 5.5 to about 9, about 5.5 to about 8, about 5.5. to about 7, about 6 to about 11, about 6 to about 10, about 6 to about 9, about 6 to to about 8, about 6 to about 7, about 6.5 to about 11, about 6.5 to about 10, about 6.5 to about 9, about 6.5 to about 8, about 6.5 to about 7, about 7 to about 11, about 7 to about 10, about 7 to about 9, or about 7 to about 8, preferably about 5.5 to about 9, and more preferably about 6 to about 9.

In particular embodiments, the low temperature washing method is conducted at a degree of hardness of from about 0°dH to about 30°dH, such as about 1°dH, about 2°dH, about 3°dH, about 4°dH, about 5°dH, about 6°dH, about 7°dH, about 8°dH, about 9°dH, about 10°dH, about 11°dH, about 12°dH, about 13°dH, about 14°dH, about 15°dH, about 16°dH, about 17°dH, about 18°dH, about 19°dH, about 20°dH, about 21°dH, about 22°dH, about 23°dH, about 24°dH, about 25°dH, about 26°dH, about 27°dH, about 28°dH, about 29°dH,about 30°dH. Under typical European wash conditions, the degree of hardness is about 15°dH, under typical US wash conditions about 6°dH, and under typical Asian wash conditions, about 3°dH.

**Animal Feed**

The present invention is also directed to methods for using the protease of the invention having protease activity in animal feed, as well as to feed compositions and feed additives comprising the proteases of the invention.

Thus one embodiment, the present invention relates to a feed composition or a feed additive comprising an isolated polypeptide having a sequence identity to the mature polypeptide of SEQ ID NO: 2 of at least 85%, which have protease activity, wherein the mature polypeptide is amino acids 189 to 374 of SEQ ID NO: 2.

Another aspect of the invention relates to a method for the treatment of proteins, comprising the step of adding at least one protease according to the invention to at least one protein or protein source such as soybean. Thus one aspect relates to a method for the treatment of proteins, comprising the step of adding at least one isolated polypeptide having a sequence identity to the mature polypeptide of SEQ ID NO: 2 of at least 85%, which have protease activity to at least one protein or protein source such as soybean, wherein the mature polypeptide is amino acids 189 to 374 of SEQ ID NO: 2.

The term animal includes all animals, including human beings. Examples of animals are non-ruminants, and ruminants. Ruminant animals include, for example, animals such as sheep, goats, and cattle, e.g. beef cattle, cows, and young calves. In a particular embodiment, the animal is a non-ruminant animal. Non-ruminant animals include mono-gastric animals, e.g. pigs or swine (including, but not limited to, piglets, growing pigs, and sows); poultry such as turkeys, ducks and chicken (including but not limited to broiler chicks, layers); horses (including but not limited to hotbloods, coldbloods and warm bloods), young calves; and fish (including but not limited to salmon, trout, tilapia, catfish and carps; and crustaceans (including but not limited to shrimps and prawns).

The term feed or feed composition means any compound, preparation, mixture, or composition suitable for, or intended for intake by an animal.

In the use according to the invention the protease can be fed to the animal before, after, or simultaneously with the diet. The latter is preferred.

In a particular embodiment, the protease, in the form in which it is added to the feed, or when being included in a feed additive, is well-defined. Well-defined means that the protease preparation is at least 50% pure as determined by Size-exclusion chromatography (see Example 12 of WO 01/58275). In other particular embodiments the protease preparation is at least 60, 70, 80, 85, 88, 90, 92, 94, or at least 95% pure as determined by this method.

A well-defined protease preparation is advantageous. For instance, it is much easier to dose correctly to the feed a protease that is essentially free from interfering or contaminating other proteases. The term dose correctly refers

in particular to the objective of obtaining consistent and constant results, and the capability of optimising dosage based upon the desired effect.

For the use in animal feed, however, the protease need not be that pure; it may e.g. include other enzymes, in which case it could be termed a protease preparation.

The protease preparation can be (a) added directly to the feed (or used directly in a protein treatment process), or (b) it can be used in the production of one or more intermediate compositions such as feed additives or premixes that is subsequently added to the feed (or used in a treatment process). The degree of purity described above refers to the purity of the original protease preparation, whether used according to (a) or (b) above.

Protease preparations with purities of this order of magnitude are in particular obtainable using recombinant methods of production, whereas they are not so easily obtained and also subject to a much higher batch-to-batch variation when the protease is produced by traditional fermentation methods.

Such protease preparation may of course be mixed with other enzymes.

The protein may be an animal protein, such as meat and bone meal, feather meal, and/or fish meal; or it may be a vegetable protein.

The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Vegetable proteins may be derived from vegetable protein sources, such as legumes and cereals, for example materials from plants of the families *Fabaceae* (*Leguminosae*), *Cruciferaceae*, *Chenopodiaceae*, and Poaceae, such as soy bean meal, lupin meal and rapeseed meal.

In a particular embodiment, the vegetable protein source is material from one or more plants of the family *Fabaceae*, e.g. soybean, lupine, pea, or bean.

In another particular embodiment, the vegetable protein source is material from one or more plants of the family *Chenopodiaceae*, e.g. beet, sugar beet, spinach or quinoa.

Other examples of vegetable protein sources are rapeseed, sunflower seed, cotton seed, and cabbage.

Soybean is a preferred vegetable protein source.

Other examples of vegetable protein sources are cereals such as barley, wheat, rye, oat, maize (corn), rice, triticale, and sorghum.

In a particular embodiment of a treatment process the protease(s) in question is affecting (or acting on, or exerting its solubilising influence on) the proteins, such as vegetable proteins or protein sources. To achieve this, the protein or protein source is typically suspended in a solvent, eg an aqueous solvent such as water, and the pH and temperature values are adjusted paying due regard to the characteristics of the enzyme in question. For example, the treatment may take place at a pH-value at which the activity of the actual protease is at least 5%, 10%, 20%, 30%, 40%, 50%, 60%, 70%, 80%, or at least 90%. Likewise, for example, the treatment may take place at a temperature at which the activity of the actual protease is at least 5%, 10%, 20%, 30%, 40%, 50%, 60%, 70%, 80%, or at least 90%. The above percentage activity indications are relative to the maximum activities. The enzymatic reaction is continued until the desired result is achieved, following which it may or may not be stopped by inactivating the enzyme, e.g. by a heat-treatment step.

In another particular embodiment of a treatment process of the invention, the protease action is sustained, meaning e.g. that the protease is added to the proteins, but its hydrolysing influence is so to speak not switched on until later when desired, once suitable hydrolysing conditions are established, or once any enzyme inhibitors are inactivated, or whatever other means could have been applied to postpone the action of the enzyme.

In one embodiment the treatment is a pre-treatment of animal feed or proteins for use in animal feed, i.e. the proteins are hydrolysed before intake.

The term improving the nutritional value of an animal feed means improving the availability of the proteins, thereby leading to increased protein extraction, higher protein yields, and/or improved protein utilisation. The nutritional value of the feed is therefore increased, also the protein and amino acid digestibility is increased and/or the growth rate and/or weight gain and/or feed conversion (i.e. the weight of ingested feed relative to weight gain) of the animal is/are improved.

The protease can be added to the feed in any form, be it as a relatively pure protease, or in admixture with other components intended for addition to animal feed, i.e. in the form of animal feed additives, such as the so-called pre-mixes for animal feed.

In a further aspect the present invention relates to compositions for use in animal feed, such as animal feed, and animal feed additives, e.g. premixes.

Apart from the protease of the invention, the animal feed additives of the invention contain at least one fat-soluble vitamin, and/or at least one water soluble vitamin, and/or at least one trace mineral, and/or at least one macro mineral.

Further, optional, feed-additive ingredients are colouring agents, e.g. carotenoids such as beta-carotene, astaxanthin, and lutein; stabilisers; growth improving additives and aroma compounds/flavorings, e.g. creosol, anethol, deca-, undeca-and/or dodeca-lactones, ionones, irone, gingerol, piperidine, propylidene phatalide, butylidene phatalide, capsaicin and/or tannin; antimicrobial peptides; polyunsaturated fatty acids (PUFAs); reactive oxygen generating species; also, a support may be used that may contain, for example, 40-50% by weight of wood fibres, 8-10% by weight of stearine, 4-5% by weight of curcuma powder, 4-58% by weight of rosemary powder, 22-28% by weight of limestone, 1-3% by weight of a gum, such as gum arabic, 5-50% by weight of sugar and/or starch and 5-15% by weight of water.
A feed or a feed additive of the invention may also comprise at least one other enzyme selected from amongst phytase (EC 3.1.3.8 or 3.1.3.26); xylanase (EC 3.2.1.8); galactanase (EC 3.2.1.89); alpha-galactosidase (EC 3.2.1.22); further protease, phospholipase A1 (EC 3.1.1.32); phospholipase A2 (EC 3.1.1.4); lysophospholipase (EC 3.1.1.5); phospholipase C (3.1.4.3); phospholipase D (EC 3.1.4.4); amylase such as, for example, alpha-amylase (EC 3.2.1.1); and/or beta-glucanase (EC 3.2.1.4 or EC 3.2.1.6).
In a particular embodiment these other enzymes are well-defined (as defined above for protease preparations).
Examples of antimicrobial peptides (AMP's) are CAP18, Leucocin A, Tritrpticin, Protegrin-1, Thanatin, Defensin, Lactoferrin, Lactoferricin, and Ovispirin such as Novispirin (Robert Lehrer, 2000), Plectasins, and Statins, including the compounds and polypeptides disclosed in WO 03/044049 and WO 03/048148, as well as variants or fragments of the above that retain antimicrobial activity.
Examples of antifungal polypeptides (AFP's) are the *Aspergillus giganteus*, and *Aspergillus niger* peptides, as well as variants and fragments thereof which retain antifungal activity, as disclosed in WO 94/01459 and WO 02/090384.
Examples of polyunsaturated fatty acids are C18, C20 and C22 polyunsaturated fatty acids, such as arachidonic acid, docosohexaenoic acid, eicosapentaenoic acid and gamma-linoleic acid.
Examples of reactive oxygen generating species are chemicals such as perborate, persulphate, or percarbonate; and enzymes such as an oxidase, an oxygenase or a syntethase.
Usally fat- and water-soluble vitamins, as well as trace minerals form part of a so-called premix intended for addition to the feed, whereas macro minerals are usually separately added to the feed. Either of these composition types, when enriched with a protease of the invention, is an animal feed additive of the invention.
In a particular embodiment, the animal feed additive of the invention is intended for being included (or prescribed as having to be included) in animal diets or feed at levels of 0.01 to 10.0%; more particularly 0.05 to 5.0%; or 0.2 to 1.0% (% meaning g additive per 100 g feed). This is so in particular for premixes.
The following are non-exclusive lists of examples of these components:

Examples of fat-soluble vitamins are vitamin A, vitamin D3, vitamin E, and vitamin K, e.g. vitamin K3.
Examples of water-soluble vitamins are vitamin B12, biotin and choline, vitamin B1, vitamin B2, vitamin B6, niacin, folic acid and panthothenate, e.g. Ca-D-panthothenate.
Examples of trace minerals are manganese, zinc, iron, copper, iodine, selenium, and cobalt.
Examples of macro minerals are calcium, phosphorus and sodium.

The nutritional requirements of these components (exemplified with poultry and piglets/pigs) are listed in Table A of WO 01/58275. Nutritional requirement means that these components should be provided in the diet in the concentrations indicated.
In the alternative, the animal feed additive of the invention comprises at least one of the individual components specified in Table A of WO 01/58275. At least one means either of, one or more of, one, or two, or three, or four and so forth up to all thirteen, or up to all fifteen individual components. More specifically, this at least one individual component is included in the additive of the invention in such an amount as to provide an in-feed-concentration within the range indicated in column four, or column five, or column six of Table A.
In a still further embodiment, the animal feed additive of the invention comprises at least one of the below vitamins, preferably to provide an in-feed-concentration within the ranges specified in the below table 1 (for piglet diets, and broiler diets, respectively).

Table 1: Typical vitamin recommendations

| Vitamin | Piglet diet | Broiler diet |
|---|---|---|
| Vitamin A | 10,000-15,000 IU/kg feed | 8-12,500 IU/kg feed |
| Vitamin D3 | 1800-2000 IU/kg feed | 3000-5000 IU/kg feed |
| Vitamin E | 60-100 mg/kg feed | 150-240 mg/kg feed |
| Vitamin K3 | 2-4 mg/kg feed | 2-4 mg/kg feed |

(continued)

| Vitamin | Piglet diet | Broiler diet |
|---|---|---|
| Vitamin B1 | 2-4 mg/kg feed | 2-3 mg/kg feed |
| Vitamin B2 | 6-10 mg/kg feed | 7-9 mg/kg feed |
| Vitamin B6 | 4-8 mg/kg feed | 3-6 mg/kg feed |
| Vitamin B12 | 0.03-0.05 mg/kg feed | 0.015-0.04 mg/kg feed |
| Niacin (Vitamin B3) | 30-50 mg/kg feed | 50-80 mg/kg feed |
| Pantothenic acid | 20-40 mg/kg feed | 10-18 mg/kg feed |
| Folic acid | 1-2 mg/kg feed | 1-2 mg/kg feed |
| Biotin | 0.15-0.4 mg/kg feed | 0.15-0.3 mg/kg feed |
| Choline chloride | 200-400 mg/kg feed | 300-600 mg/kg feed |

The present invention also relates to animal feed compositions. Animal feed compositions or diets have a relatively high content of protein. Poultry and pig diets can be characterised as indicated in Table B of WO 01/58275, columns 2-3. Fish diets can be characterised as indicated in column 4 of this Table B. Furthermore such fish diets usually have a crude fat content of 200-310 g/kg.

WO 01/58275 corresponds to US 09/779334.

An animal feed composition according to the invention has a crude protein content of 50-800 g/kg, and furthermore comprises at least one protease as claimed herein.

Furthermore, or in the alternative (to the crude protein content indicated above), the animal feed composition of the invention has a content of metabolisable energy of 10-30 MJ/kg; and/or a content of calcium of 0.1-200 g/kg; and/or a content of available phosphorus of 0.1-200 g/kg; and/or a content of methionine of 0.1-100 g/kg; and/or a content of methionine plus cysteine of 0.1-150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 in Table B of WO 01/58275 (R. 2-5).

Crude protein is calculated as nitrogen (N) multiplied by a factor 6.25, i.e. Crude protein (g/kg)= N (g/kg) x 6.25. The nitrogen content is determined by the Kjeldahl method (A.O.A.C., 1984, Official Methods of Analysis 14th ed., Association of Official Analytical Chemists, Washington DC).

Metabolisable energy can be calculated on the basis of the NRC publication Nutrient requirements in swine, ninth revised edition 1988, subcommittee on swine nutrition, committee on animal nutrition, board of agriculture, national research council. National Academy Press, Washington, D.C., pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

The dietary content of calcium, available phosphorus and amino acids in complete animal diets is calculated on the basis of feed tables such as Veevoedertabel 1997, gegevens over chemische samenstelling, verteerbaarheid en voederwaarde van voedermiddelen, Central Veevoederbureau, Runderweg 6, 8219 pk Lelystad. ISBN 90-72839-13-7.

In a particular embodiment, the animal feed composition of the invention contains at least one vegetable protein as defined above.

The animal feed composition of the invention may also contain animal protein, such as Meat and Bone Meal, Feather meal, and/or Fish Meal, typically in an amount of 0-25%. The animal feed composition of the invention may also comprise Dried Destillers Grains with Solubles (DDGS), typically in amounts of 0-30%.

In still further particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-25% fish meal; and/or 0-25% meat and bone meal; and/or 0-20% whey.

Animal diets can e.g. be manufactured as mash feed (non pelleted) or pelleted feed. Typically, the milled feed-stuffs are mixed and sufficient amounts of essential vitamins and minerals are added according to the specifications for the species in question. Enzymes can be added as solid or liquid enzyme formulations. For example, for mash feed a solid or liquid enzyme formulation may be added before or during the ingredient mixing step. For pelleted feed the (liquid or solid) protease/enzyme preparation may also be added before or during the feed ingredient step. Typically a liquid protease/enzyme preparation is added after the pelleting step. The enzyme may also be incorporated in a feed additive or premix.

The final enzyme concentration in the diet is within the range of 0.01-200 mg enzyme protein per kg diet, for

example in the range of 0.5-25 mg enzyme protein per kg animal diet.

The protease should of course be applied in an effective amount, i.e. in an amount adequate for improving hydrolysis, digestibility, and/or improving nutritional value of feed. It is at present contemplated that the enzyme is administered in one or more of the following amounts (dosage ranges): 0.01-200; 0.01-100; 0.5-100; 1-50; 5-100; 10-100; 0.05-50; or 0.10-10 - all these ranges being in mg protease protein per kg feed (ppm).

For determining mg protease protein per kg feed, the protease is purified from the feed composition, and the specific activity of the purified protease is determined using a relevant assay (see under protease activity, substrates, and assays). The protease activity of the feed composition as such is also determined using the same assay, and on the basis of these two determinations, the dosage in mg protease protein per kg feed is calculated.

The same principles apply for determining mg protease protein in feed additives. Of course, if a sample is available of the protease used for preparing the feed additive or the feed, the specific activity is determined from this sample (no need to purify the protease from the feed composition or the additive).

The present invention is further described by the following examples that should not be construed as limiting the scope of the invention.

**Materials and Methods**

**Wash assays**

Automatic Mechanical Stress Assay (AMSA) for laundry

In order to assess the wash performance in laundry washing experiments are performed, using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the laundry sample, the textile to be washed against all the slot openings. During the washing time, the plate, test solutions, textile and lid are vigorously shaken to bring the test solution in contact with the textile and apply mechanical stress in a regular, periodic oscillating manner. For further description see WO02/42740 especially the paragraph "Special method embodiments" at page 23-24.

The wash performance is measured as the brightness of the colour of the textile washed. Brightness can also be expressed as the intensity of the light reflected from the sample when illuminated with white light. When the sample is stained the intensity of the reflected light is lower, than that of a clean sample. Therefore the intensity of the reflected light can be used to measure wash performance.

Colour measurements are made with a professional flatbed scanner (Kodak iQsmart, Kodak, Midtager 29, DK-2605 Brøndby, Denmark), which is used to capture an image of the washed textile.

To extract a value for the light intensity from the scanned images, 24-bit pixel values from the image are converted into values for red, green and blue (RGB). The intensity value (Int) is calculated by adding the RGB values together as vectors and then taking the length of the resulting vector:

$$Int = \sqrt{r^2 + g^2 + b^2}.$$

Table 2: Composition of model detergents and test materials

| | |
|---|---|
| Laundry powder model detergent A | Sodium citrate dihydrate 32.3%<br>Sodium-LAS 24.2%<br>Sodium lauryl sulfate 32.2%<br>Neodol 25-7 (alcohol ethoxylate) 6.4%<br>Sodium sulfate 4.9% |

(continued)

| | |
|---|---|
| Laundry liquid model detergent B | Water 30.63%<br>Sodium hydroxide 2.95%<br>Dodecylbenzensulfonic acid 11.52%<br>Fatty acids (Soya) 5.50%<br>Propane-1,2-diol (MPG) 5.05%<br>Water 17.38%<br>C13-alcohol ethoxylate, 10.50%<br>Diethylenetriaminepentakis (methylenephosphonic acid) (DTMPA) 3.08%<br>Triethanolamine (TEA) 2.22%<br>Fatty acids (Coco) 4.50%<br>Sodium citrate monohydrate 1.00%<br>Ethanol 4.63%<br>Syntran 5909 (opacifier) 0.30%<br>Perfume 0.35% |
| Test material | PC-03 (Chocolate-milk/ink on cotton/polyester)<br>C-10 (Oil/milk/pigment on cotton)<br>PC-05 (Blood/milk/ink on cotton/polyester)<br>EMPA117EH (Blood/milk/ink on cotton/polyester)<br>CS-37, Full egg with pigment non-aged on cotton |
| Test materials are obtained from Center For Testmaterials BV, P.O. Box 120, 3133 KT Vlaardingen, the Netherlands and EMPA Testmaterials AG, Movenstrasse 12, CH-9015 St. Gallen, Switzerland. | |

**Protease assays**

A kinetic Suc-AAPF-pNA assay was used for obtaining the pH-activity profile and the pH-stability profile and the inhibition at pH 9.

A Protazyme AK (cross-linked and dyed casein) assay was used for obtaining the temperature-activity profile at pH 6.5 and at pH 9.

1) Suc-AAPF-pNA assay:

| | |
|---|---|
| pNA substrate : | Suc-AAPF-pNA (Bachem L-1400). |
| Temperature : | Room temperature (25°C) |
| Assay buffers : | 100mM succinic acid, 100mM HEPES, 100mM CHES, 100mM CABS, 1 mM $CaCl_2$, 150mM KCl, 0.01% Triton X-100 adjusted to pH-values 2.0, 3.0, 4.0, 5.0, 6.0, 7.0, 8.0, 9.0, 10.0, and 11.0 with HCl or NaOH. |

μl protease (diluted in 0.01% Triton X-100) was mixed with 100μl assay buffer. The assay was started by adding 100μl pNA substrate (50mg dissolved in 1.0ml DMSO and further diluted 45x with 0.01% Triton X-100). The increase in $OD_{405}$ was monitored as a measure of the protease activity.

2) Protazyme AK assay:

| | |
|---|---|
| Substrate | : Protazyme AK tablet (cross-linked and dyed casein; from Megazyme) |
| Temperature : | controlled (assay temperature). |
| Assay buffer : | 100mM succinic acid, 100mM HEPES, 100mM CHES, 100mM CABS, 1 mM $CaCl_2$, 150mM KCl, 0.01% Triton X-100, pH 7.0. |

A Protazyme AK tablet was suspended in 2.0 ml 0.01% Triton X-100 by gentle stirring. 500μl of this suspension and 500μl assay buffer were dispensed in an Eppendorf tube and placed on ice. μl protease sample (diluted in 0.01% Triton X-100) was added. The assay was initiated by transferring the Eppendorf tube to an Eppendorf thermomixer, which was set to the assay temperature. The tube was incubated for 15 minutes on the Eppendorf thermomixer at its highest shaking rate (1400 rpm.). The incubation was stopped by transferring the tube back to the ice bath. Then the tube was centrifuged in an ice cold centrifuge for a few minutes and 200μl supernatant was transferred to a microtiter plate. $OD_{650}$ was read as a measure of protease activity. A buffer blind was included in the assay (instead of enzyme).

3) Suc-AAPX-pNA assay:

| | |
|---|---|
| pNA substrates: | Suc-AAPA-pNA (Bachem L-1775)<br>Suc-AAPR-pNA (Bachem L-1720)<br>Suc-AAPD-pNA (Bachem L-1835)<br>Suc-AAPI-pNA (Bachem L-1790)<br>Suc-AAPM-pNA (Bachem L-1395)<br>Suc-AAPV-pNA (Bachem L-1770)<br>Suc-AAPL-pNA (Bachem L-1390)<br>Suc-AAPE-pNA (Bachem L-1710)<br>Suc-AAPK-pNA (Bachem L-1725)<br>Suc-AAPF-pNA (Bachem L-1400) |
| Temperature : | Room temperature (25°C) |
| Assay buffer : | 100mM succinic acid, 100mM HEPES, 100mM CHES, 100mM CABS, 1 mM $CaCl_2$, 150mM KCl, 0.01% Triton X-100, pH 9.0. |

20μl protease (diluted in 0.01% Triton X-100) was mixed with 100μl assay buffer. The assay was started by adding 100μl pNA substrate (50mg dissolved in 1.0 ml DMSO and further diluted 45x with 0.01% Triton X-100). The increase in $OD_{405}$ was monitored as a measure of the protease activity.

**Soybean-Maize Meal Assay (SMM Assay)**

**Protease activity on soybean-maize meal at pH 3, 4, 5, 6, and 7**

An end-point assay using soybean-maize meal as substrate was used for obtaining the activity profile of the proteases at pH 3-7.

Assay buffers: 100 mM succinic acid, 100 mM HEPES, 100 mM CHES, 100 mM CAPS, 1 mM $CaCl_2$, 150 mM KCl, 0.01% Triton X-100 adjusted using HCl or NaOH to pH-values 3.0, 4.0, 5.0, 6.0, 7.0, 8.0, 9.0, 10.0 and 11.0 when mixing 10 ml assay buffer with 1 g soybean-maize meal (30:70 ratio).

2 mL soybean-maize meal slurry is mixed for 30 min before protease addition and incubation for 3 hours at 40°C (500 rpm). Protease is added via 100 μl 100 mM sodium acetate (NaAc) buffer (9.565 g/l NaAc, 1.75 g/l acetic acid, 5 mM $CaCl_2$, 0.01 % BSA, 0.01 % Tween20, pH 6.0). Supernatant are collected after centrifugation (10 min, 4000 rpm, 0°C) and protein activity is determined using a colorimetric assay based on the o-phthat-dialdehyde (OPA) method essentially according to Nielsen et al. (Nielsen, PM, Petersen, D, Dampmann, C. Improved method for determining food protein degree of hydrolysis. J Food Sci, 2001, 66: 642-646). This assay detects free α-amino groups and hence protease activity can be measured as an increase in absorbance. First 500 μl of each supernatant is filtered through a 100 kDa Microcon filter by centrifugation (60 min, 11,000 rpm, 5°C). The samples are diluted 10x in deionized water and 25 μl of each sample is loaded into a 96 well microtiter plate (5 replicates). Finally 200 μl OPA reagent is dispensed into all wells and the plate is shaken (10 sec, 750 rpm) and absorbance measured at 340 nm. The level of protease activity is calculated as the difference between absorbance in the enzyme treated sample and the blank sample and expressed as 'OD x dilution factor'.

**In vitro digestion assay**

An in vitro digestion assay was used to evaluate the effect of the proteases on a feed substrate (soybean-maize meal) in a setup designed to simulate digestion in monogastric animals. The incubation process consisted of a gastric

digestion phase with porcine pepsin (SP7000, Sigma-Aldrich, St. Louis, MO, USA) at pH 3 followed by a short duodenal incubation at pH 3.8 and a small intestinal incubation with pancreatin (8xUSB, P-7545, Sigma-Aldrich, St. Louis, MO, USA) at pH 7.0.

The *in vitro* digestion was performed using an automated system based on a Gilson liquid handler (Biolab, Denmark). For each sample 0.8 g feed was weighed into a tube and all tubes were placed in the liquid handler (40°C, 500 rpm). Additions of solutions as well as pH measurements were performed automatically. At time 0 min, 4.1 mL HCl (24 mM $CaCl_2$) was added to reach pH 3.0 in the solution. At time 30 min 0.5 ml HCl (24 mM $CaCl_2$, 3000 U pepsin/g feed) and 100 μL of a 100 mM sodium acetate buffer (258.6 g NaAc per litre, 0.57 % acetic acid, pH 6.0) was added. At time 90 min 900 μL NaOH was added to reach pH ~3.8 and at time 120 min 400 μL of a 1 M $NaHCO_3$ solution containing 6.5 mg pancreatin/g feed was added leading to pH 6.8 in the solution. The pH was measured at time 30, 60, 90, 115, 120 and 180 min. The test proteases were added via the 100 μl NaAc buffer at time 30 min.

The degree of protein hydrolysis (DH) was determined using a colorimetric assay based on the o-phthatdialdehyde (OPA) method essentially according to Nielsen et al. (Nielsen, PM, Petersen, D, Dampmann, C. Improved method for determining food protein degree of hydrolysis. J Food Sci, 2001, 66: 642-646). This assay detects free α-amino groups and hence protease activity can be measured as an increase in absorbance. First 500 μl of each supernatant is filtered through a 100 kDa Microcon filter by centrifugation (60 min, 11,000 rpm, 5°C). The samples are diluted 100x in deionized water and 25 μl of each sample is loaded into a 96 well microtiter plate (5 replicates). Finally 200 μl OPA reagent is dispensed into all wells and the plate is shaken (10 sec, 750 rpm) and absorbance measured at 340 nm. The percentage of cleaved peptide bonds (DH) was calculated as:

$$DH\ (\%) = 100 \times h / h_{tot},$$

where $h_{tot}$ is the total number of peptide bonds per protein equivalent, and h is the number of hydrolyzed bonds. Calculation of $h_{tot}$ is based on the amino acid sequence of the raw material. In this study the value for soy was used (7.8 g equivalents per kg protein) according to Adler-Nissen (1986). The expression for h in the OPA method is:

$$h = (\text{serine-NH}_2 - \beta) / \alpha \text{ meqv/g protein},$$

where $\alpha$ = 0.970 and $\beta$ = 0.342 according to Adler-Nissen (1979). Serine-$NH_2$ is calculated as:

$$\text{Serine - NH}_2 = (OD_{blank} - OD_{sample}) / (OD_{standard} - OD_{blank}) \times 0.9516 \text{ meqv/L} \times 0.1 \times 100 / X \times P,$$

where serine-$NH_2$ = meqv serine-$NH_2$/g protein; X = g sample; P = protein % in sample and 0.1 is the sample volume in litres (L).

Statistics: Statistical analysis of the parameters registered was performed using an analysis of variance (ANOVA) procedure and comparison of means was done using the Student t-test ($\alpha$ = 0.05) provided by the ANOVA procedure (SAS, JMP® 5 Administrators Guide to Annually Licensed Windows, Mackintosh, and Linux Versions, Release 5.1. SAS Institute, Cary, NC. (2003)).

## EXAMPLES

### Strains

*Saccharothrix australiensis* DSM 43800, Available from Deutsche Sammlung von Microorganismen und Zellkulturen GmbH, Braunschweig, Germany. The strain was originally collected from soil in Australia.

### Media and Solutions

LB plates were composed of 10 g of Bacto-Tryptone, 5 g of yeast extract, 10 g of sodium chloride, 15 g of Bacto-agar, and deionized water to 1 liter.

LB medium was composed of 10 g of Bacto-Tryptone, 5 g of yeast extract, and 10 g of sodium chloride, and deionized water to 1 liter.

**Example 1: DNA-preparation and sequencing of the** *Saccharothrix australiensis* **genome**

Chromosomal DNA *Saccharothrix australiensis* was isolated by QIAamp DNA Blood Mini Kit " (Qiagen, Hilden, Germany). 5ug of chromosomal DNA of each strain were sent for genome sequencing at FASTERIS SA, Switzerland. The genomes were sequenced by Illumina Sequencing. The genome sequences were analysed for secreted S1 proteases and the S1 protease (SEQ ID:1/SEQ ID:2) was identified.

**Example 2: Expression of the S1 protease from *Saccharothrix australiensis***

Based on the nucleotide sequence identified as SEQ ID NO: 1, a synthetic gene having SEQ ID NO: 3, was synthesized by Gene Art (GENEART AG BioPark, Josef-Engert-Str. 11, 93053, Regensburg, Germany). The synthetic gene was subcloned using ClaI and MluI restriction sites into Bacillus expression vector as described in PCT/EP2011/064585 Example 1. Transformants were selected on LB plates supplemented with 6 $\mu$g of chloramphenicol per ml. The recombinant *Bacillus subtilis* clone containing the integrated expression construct was selected and cultivated on a rotary shaking table in 500 mL baffled Erlenmeyer flasks each containing 100 ml casein-based media supplemented with 34 mg/l chloramphenicol. The clone was cultivated for 5 days at 30°C. The enzyme containing supernatants were harvested and the enzyme purified as described in example 3.

**Example 3: Purification of the S1 protease from *Saccharothrix australiensis***

The culture broth from example 2 was centrifuged (20000 x g, 20 min) and the supernatant was carefully decanted from the precipitate. The supernatant was filtered through a Nalgene 0.2$\mu$m filtration unit in order to remove the rest of the *Bacillus* host cells. The 0.2$\mu$m filtrate was transferred to 50mM $H_3BO_3$, 20mM $CH_3COOH$/NaOH, 1 mM $CaCl_2$, pH 4.5 on a G25 Sephadex column (from GE Healthcare). The G25 sephadex transferred enzyme was applied to a SP-sepharose FF column (from GE Healthcare) equilibrated in 50mM $H_3BO_3$, 20mM $CH_3COOH$/NaOH, 1mM $CaCl_2$, pH 4.5. After washing the column extensively with the equilibration buffer, the protease was eluted with a linear NaCl gradient (0 $\rightarrow$ 0.5M) in the same buffer over five column volumes. Fractions from the column were analysed for protease activity (using the Suc-AAPF-pNA assay at pH 9). The protease peak was pooled and diluted ten times with deionised water to reduce the conductivity of the sample and was applied to a SOURCE S column (from GE Healthcare) equilibrated in 50mM $H_3BO_3$, 20mM $CH_3COOH$/NaOH, 1mM $CaCl_2$, pH 4.5. After washing the column extensively with the equilibration buffer, the protease was eluted with a linear NaCl gradient (0 $\rightarrow$ 0.5M) in the same buffer over ten column volumes. Fractions from the column were analysed for protease activity (using the Suc-AAPF-pNA assay at pH 9) and active fractions were further analysed by SDS-PAGE. Fractions where only one band was seen on the coomassie stained SDS-PAGE gel, were pooled as the purified product and was used for further characterization.

**Example 4: Characterization of the S1 protease from *Saccharothrix australiensis***

The Suc-AAPF-pNA assay was used for obtaining the pH-activity profile and the pH-stability profile (residual activity after 2 hours at indicated pH-values). For the pH-stability profile the protease was diluted 10x in the different Assay buffers to reach the pH-values of these buffers and then incubated for 2 hours at 37°C. After incubation, the pH of the protease incubations was transferred to the same pH-value, before assay for residual activity, by dilution in the pH 9.0 Assay buffer. The Protazyme AK assay was used for obtaining the temperature-activity profile at pH 7.0. The Suc-AAPX-pNA assay and ten different Suc-AAPX-pNA substrates were used for obtaining the P1-specificity of the enzymes at pH 9.0.

The results are shown in Tables 3-6 below. The results are compared with the Protease 10R (SEQ ID NO 7). For Table 3, the activities are relative to the optimal pH for the enzyme. For Table 4, the activities are residual activities relative to a sample, which was kept at stable conditions (5°C, pH 9.0). For Table 5, the activities are relative to the optimal temperature at pH 7.0 or pH 6.5 for the enzyme. For Table 6, the activities are relative to the best substrate (Suc-AAPF-pNA) for the enzyme.

Table 3: pH-activity profile

| pH | S1 Protease from *Saccharothrix australiensis* | Protease 10R |
|---|---|---|
| 2 | 0.00 | - |
| 3 | 0.01 | 0.00 |
| 4 | 0.03 | 0.02 |

(continued)

| pH | S1 Protease from *Saccharothrix australiensis* | Protease 10R |
|---|---|---|
| 5 | 0.09 | 0.07 |
| 6 | 0.24 | 0.21 |
| 7 | 0.45 | 0.44 |
| 8 | 0.73 | 0.67 |
| 9 | 0.92 | 0.88 |
| 10 | 0.97 | 1.00 |
| 11 | 1.00 | 0.93 |

Table 4: pH-stability profile (residual activity after 2 hours at 37°C)

| pH | S1 Protease from *Saccharothrix australiensis* | Protease 10R |
|---|---|---|
| 2 | 0.58 | 0.78 |
| 3 | 1.02 | 1.03 |
| 4 | 0.99 | 0.99 |
| 5 | 0.99 | 1.00 |
| 6 | 1.00 | 1.03 |
| 7 | 1.01 | 1.01 |
| 8 | 1.03 | 0.98 |
| 9 | 0.98 | 0.99 |
| 10 | 0.94 | 0.99 |
| 11 | 0.90 | 0.86 |
| After 2 hours at 5 °C | 1.00 (at pH 9) | 1.00 (at pH 9) |

Table 5: Temperature activity profile

| Temp (°C) | S1 protease from *Saccharothrix australiensis* (pH 7) | Protease 10R (pH 6.5) |
|---|---|---|
| 15 | 0.02 | 0.01 |
| 25 | 0.06 | 0.02 |
| 37 | 0.14 | 0.06 |
| 50 | 0.37 | 0.13 |
| 60 | 0.71 | 0.35 |
| 70 | 1.00 | 0.96 |
| 80 | 0.22 | 1.00 |

Table 6: P1-specificity on 10 Suc-AAPX-pNA substrates at pH 9.0

| Suc-AAPX-pNA | S1 Protease from *Saccharothrix australiensis* | Protease 10R (pH 9) |
|---|---|---|
| Suc-AAPA-pNA | 0.08 | 0.13 |
| Suc-AAPR-pNA | 0.06 | 0.09 |
| Suc-AAPD-pNA | 0.00 | 0.00 |

(continued)

| Suc-AAPX-pNA | S1 Protease from *Saccharothrix australiensis* | Protease 10R (pH 9) |
|---|---|---|
| Suc-AAPI-pNA | 0.00 | 0.00 |
| Suc-AAPM-pNA | 0.47 | 0.78 |
| Suc-AAPV-pNA | 0.00 | 0.01 |
| Suc-AAPL-pNA | 0.18 | 0.18 |
| Suc-AAPE-pNA | 0.00 | 0.00 |
| Suc-AAPK-pNA | 0.06 | 0.08 |
| Suc-AAPF-pNA | 1.00 | 1.00 |

Other characteristics

The S1 protease from *Saccharothrix australiensis* was inhibited by PMSF.
Determination of the N-terminal sequence was: IDVIGGN (SEQ ID NO: 4).
The relative molecular weight as determined by SDS-PAGE was approx. $M_r$ = 21 kDa.
The molecular weight determined by Intact molecular weight analysis was 18746.9Da.
The mature sequence (from MS-EDMAN data):

IDVIGGNAYYMGSGGRCSVGFSVNGGFVTAGHCGRVGTTTTQPSGTFAGSTFPGRDYAWV
RVSSGNTMRGLVNRYPGTVPVKGSNESSVGASVCRSGSTTGWHCGTIQQKNTSVTYPEGT
ISGVTRTNACAEPGDSGGSWLTGDQAQGVTSGGSGNCSSGGTTYFQPVNPILQAYGLQLV
IEGGPT (SEQ ID NO: 5)

The calculated molecular weight from this mature sequence was 18746.5Da.

**Example 5: AMSA wash using the S1 protease from *Saccharothrix australiensis***

The wash performance of S1 protease from *Saccharothrix australiensis* was tested using two different liquid detergents and a powder detergent at 2 different wash temperatures on 4 different technical stains using the Automatic Mechanical Stress Assay.

The experiments were conducted as described in the AMSA for laundry method using a single cycle wash procedure, with the detergent composition and swatches described in table 2 and the experimental conditions as specified in table 7 below.

Table 7: Experimental conditions for laundry experiments

| | |
|---|---|
| Detergent dosage | powder model detergent A 2.5 g/L, liquid model detergent B 2 g/L & 8 g/L or Unilever Persil Small&Mighty 1.33 g/L |
| Test solution volume | 160 micro L |
| pH | As is |
| Wash time | 20 minutes |
| Temperature | 20°C or 40°C |
| Water hardness | 15°dH |
| Protease concentration | 30 nM |
| Swatch | PC-05, PC-03, CS-37, C-10 |

Water hardness was adjusted to 15°dH by addition of $CaCl_2$, $MgCl_2$, and $NaHCO_3$ ($Ca^{2+}$:$Mg^{2+}$:$CO_3^-$ = 4:1:7.5)

to the test system. After washing the textiles were flushed in tap water and dried.

Table 8: Delta intensity value of detergent containing S1 protease from *Saccharothrix australiensis* compared to detergent without protease at 20°C.

| Swatch | Detergent A (2.5 g/L) at 20°C | Small&Mighty, Persil, Unilever (1.33 g/L) at 20°C | Detergent B (2 g/L) at 20°C | Detergent B (8 g/L) at 20°C |
|---|---|---|---|---|
| PC-03 | 1 | 0 | 1 | 1 |
| C-10 | 3 | 2 | 0 | 0 |
| PC-05 | 12 | 3 | 5 | 9 |
| CS-37 | 6 | 7 | 7 | 6 |

The results show that detergent containing S1 protease from *Saccharothrix australiensis* is more effective at removing stains compared to detergent without protease. The S1 protease from *Saccharothrix australiensis* is effective at removing blood and egg stains at 20°C.

Table 9: Delta intensity value of detergent containing S1 protease from *Saccharothrix australiensis* compared to detergent without protease at 40°C.

| Swatch | Detergent A (2.5 g/L) at 40°C | Small&Mighty, Persil, Unilever (1.33 g/L) at 40°C | Detergent B (2 g/L) at 40°C | Detergent B (8 g/L) at 40°C |
|---|---|---|---|---|
| PC-03 | 7 | 3 | 3 | 8 |
| C-10 | 4 | 10 | 8 | 9 |
| PC-05 | 50 | 17 | 12 | 36 |
| CS-37 | 6 | 15 | 9 | 7 |

The results show that detergent containing S1 protease from *Saccharothrix australiensis* is more effective at removing stains compared to detergent without protease. S1 protease from *Saccharothrix australiensis* is effective at removing blood, egg and milk stains at 40°C.

**Example 6: Evaluation of the Stability of S1 protease from *Saccharothrix australiensis* in liquid detergent using AMSA**

The stability of the S1 protease from *Saccharothrix australiensis* in detergent was tested by examining the wash performance of the detergent with protease using an Automatic Mechanical Stress Assay at 2 different wash temperatures. Three different stability conditions were tested, which are:

the protease was added to the detergent composition immediately before wash;
the protease was pre-incubated with the detergent for 48 hours at 25°C; and
the protease was pre-incubated in wash liquor for 30 minutes at 40°C before starting the wash.

The experiments were conducted as described in the Automatic Mechanical Stress Assay (AMSA) for laundry method using a single cycle wash procedure, with the detergent composition and swatches described in table 2 and the experimental conditions as specified in table 10 below.

Table 10: Experimental conditions for AMSA for table 11

| | |
|---|---|
| Test solution | 8 g/L liquid model detergent B |
| Test solution volume | 160 micro L |
| pH | As is |
| Wash time | 20 minutes |
| Temperature | 20°C or 40°C |

(continued)

| | |
|---|---|
| Water hardness | 15°dH |
| Protease concentration | 0 (blank) or 30 nM |
| Swatch | PC-05 |

Water hardness was adjusted to 15°dH by addition of $CaCl_2$, $MgCl_2$, and $NaHCO_3$ ($Ca^{2+}$:$Mg^{2+}$:$CO_3^-$ = 4:1:7.5) to the test system. After washing the textiles were flushed in tap water and dried.

Table 11: Delta intensity value of detergent containing S1 protease from *Saccharothrix australiensis* compared to detergent without protease on a PC-05 swatch

| | **Wash performance at 20°C** | | | **Wash performance at 40°C** | | |
|---|---|---|---|---|---|---|
| | **Fresh enzyme** | **½-hr pre incubation at 40°C** | **48hr in-detergent stability at 25°C** | **Fresh enzyme** | **½-hr pre incubation at 40°C** | **48hr in-detergent stability at 25°C** |
| *Saccharothrix australiensis* | 108 | 105 | 88 | 92 | 94 | 73 |
| *Savinase* (*SEQ ID NO 6*) | 83 | 74 | 80 | 78 | 77 | 73 |

The results show that detergent containing S1 protease from *Saccharothrix australiensis* has the same wash performance after 48 hours storage at 25°C in liquid detergent as the fresh enzyme which is added to the detergent immediately prior to the wash. This shows that under these conditions the S1 protease from *Saccharothrix australiensis* shows detergent stability.

Moreover, the results show that detergent containing S1 protease from *Saccharothrix australiensis* has the same wash performance after a 30 minutes pre-incubation of the wash liquor at 40°C as wash liquor prepared with fresh enzyme added to the detergent immediately prior to the wash. This shows that under these conditions the S1 protease from *Saccharothrix australiensis* shows in-wash stability.

**Example 7: Protease activity on soybean-maize meal assay**

Results from performing the above mentioned Soybean-maize meal assay are shown in Table 12 below. The proteolytic activity of the S1 protease from *Saccharothrix australiensis* on soybean-maize meal increases with increasing pH from pH 3 to pH 7, and the activity at pH 6-7 is as high as for protease 10R (protease derived from *Nocardiopsis sp.* strain 10R, Disclosed in WO 88/03947) indicating that the S1 protease from *Saccharothrix australiensis* might have the same effect on protein hydrolysis in the intestine of pigs and poultry where pH is around 7 as this commercially product.

Table 12: Protease activity on soybean-maize meal at pH 3.0, 4.0, 5.0, 6.0 and 7.0

| pH | *S1 Protease from Saccharothrix australiensis* | | Protease 10R | |
|---|---|---|---|---|
| | Average | Standard deviation | Average | Standard deviation |
| 3.0 | 0.18 | 0.02 | 0.22 | 0.06 |
| 4.0 | 0.29 | 0.05 | 0.30 | 0.10 |
| 5.0 | 0.58 | 0.11 | 0.71 | 0.01 |
| 6.0 | 1.81 | 0.03 | 1.81 | 0.14 |
| 7.0 | 2.65 | 0.00 | 2.92 | 0.11 |

Figure 1 shows the activity on soybean-maize meal of the S1 protease from *Saccharothrix australiensis* compared to the 10R protease.

**Example 8: In vitro digestion assay**

An in vitro digestion assay was performed as described above where the S1 protease from *Saccharothrix australiensis* was compared with protease 10R.

The results are shown in Table 13 below. The S1 protease from *Saccharothrix australiensis* increased the degree of protein hydrolysis in the samples to the same extent as protease 10R indicating that the two proteases are as efficient at hydrolyising protein present in a soy-maize diet.

Table 13: The degree of protein hydrolysis (DH) as percent in *in vitro* digestion samples after treatment with S1 protease from *Saccharothrix australiensis* or protease 10R

| Enzyme (mg enzyme protein/kg feed) | DH (%) | |
|---|---|---|
| | Average 1 | Standard deviation |
| No enzyme | $30.15^{b}$ | 0.51 |
| S1 protease from *Saccharothrix australiensis* (100) | $31.81^{ab}$ | 0.75 |
| Protease 10R (100) | $32.39^{a}$ | 1.29 |
| [1] Different superscript letters indicate significant differences (P<0.05). | | |

**Example 9: Proteolytic activity on crop, gizzard and ileum digesta from broiler chickens**

Crop, gizzard and ileum digesta material from 21 day old broiler chickens fed a corn-soy diet was collected; freeze dried and ground using a small coffee mill. The ground samples were suspended (47% w/v) in the following buffers and left to hydrate at 4°C over night (no stirring):

| | |
|---|---|
| Crop buffer: | 100 mM HEPES, 1 mM $CaCl_2 \cdot 2\ H_2O$, 150 mM KCl, 0.01% Triton X-100, adjusted to pH 5 using HCl |
| Gizzard buffer: | 100 mM succinic acid, 1 mM $CaCl_2 \cdot 2\ H_2O$, 150 mM KCl, 0.01% Triton X-100, adjusted to pH 1.67 using HCl |
| Ileum buffer: | 100 mM HEPES, 1 mM $CaCl_2 \cdot 2\ H_2O$, 150 mM KCl, 0.01% Triton X-100, adjusted to pH 7.2 using HCl |

The resulting pH was: pH 5 in crop samples; pH 3 in gizzard samples; and pH 7 in ileum samples. The suspensions were heated to 40°C and 1 ml was dispensed into tubes kept at 40°C. Three tubes representing blank ($T_0$) were immediately centrifuged (3000 x g, 0°C, 10 min) and the supernatants frozen. Either enzyme (200 mg enzyme protein/kg substrate) in 50 μl 100 mM sodium acetate buffer (9.565 g/l NaOAc, 1.75 g/l acetic acid, 5 mM $CaCl_2$, 0.01% BSA, 0.01% Tween20, pH 6.0) or just sodium acetate buffer (50 μL) for the blank samples was added to the tubes and crop and ileum samples were incubated for 3 hours ($T_3$) while the gizzard samples were incubated for 1 hour ($T_1$) at 40°C while shaking (500 rpm). The samples were centrifuged (3000 x g, 0°C, 10 min) and supernatants recovered and frozen. The proteolytic activity was determined by analyzing primary amines using the o-phthaldialdehyde (OPA) assay. The results are shown in Table 14. For each of the digesta types (crop, gizzard and ileum) there was a significant difference between the level of soluble primary amines in the blank To sample and the blank samples incubated for 1 or 3 hours. This difference may be ascribed to solubilisation and activity of proteases present in the substrate and originating from either the diet raw materials or the animal. The S1 protease from *Saccharothrix australiensis* numerically increased the level of soluble primary amines in all three assays compared to the blank incubated for 1 or 3 hours without protease.

**Table 14: Proteolytic activity of the S1 protease from *Saccharothrix australiensis* compared to Protease 10R when incubated with broiler digesta and expressed as level of primary amines measured by the OPA assay ($OD_{340}$ x dilution factor)**

| Treatment | Crop (3 hours) | Gizzard (1 hour) | Ileum (3 hours) |
|---|---|---|---|
| Blank ($T_0$) | $2.21 \pm 0.02^{d}$ | $2.95 \pm 0.02^{c}$ | $9.37 \pm 0.08^{b}$ |
| Blank | $3.54 \pm 0.02^{c}$ | $3.85 \pm 0.13^{a}$ | $14.42 \pm 0.52^{a}$ |

(continued)

| Treatment | Crop (3 hours) | Gizzard (1 hour) | Ileum (3 hours) |
|---|---|---|---|
| Protease 10R | 3.85 ± 0.07[ab] | 3.87 ± 0.21[a] | 14.74 ± 0.16[a] |
| S1 protease from *Saccharothrix australiensis* | 3.73 ± 0.08[abc] | 3.79 ± 0.12[ab] | 14.01 ± 0.58[a] |
| [a,b,c,d] Values within a column that are not connected by the same superscript letters are statistically different as determined by the Tukey Kramer test ($\alpha$=0.05) provided by the ANOVA procedure (SAS Institute Inc.). | | | |

## Claims

1. An isolated polypeptide having protease activity, selected from the group consisting of:

   (a) a polypeptide having at least 85% sequence identity to the mature polypeptide of SEQ ID NO: 2, wherein the mature polypeptide is amino acids 189 to 374 of SEQ ID NO: 2;
   (b) a polypeptide encoded by a polynucleotide having at least 85% sequence identity to the mature polypeptide coding sequence of SEQ ID NO: 1, wherein the mature polynucleotide is nucleotides 665 to 1222 of SEQ ID NO: 1; and
   (c) a fragment of the polypeptide of (a) or (b) that has protease activity,

   wherein the polypeptide has improved protease activity between 37°C and 60°C, compared to protease 10R (SEQ ID NO: 7).

2. The polypeptide of claim 1, comprising or consisting of SEQ ID NO: 2 or the mature polypeptide of SEQ ID NO: 2.

3. The polypeptide of any of claims 1-2, which is a variant of the mature polypeptide of SEQ ID NO: 2 comprising a substitution, deletion, and/or insertion at one or more positions.

4. A composition comprising the polypeptide of any of claims 1-3.

5. The composition of claim 4 being a detergent composition such as a composition for laundry or automatic dish washing.

6. The composition of claim 5 further comprising one or more additional enzymes selected among proteases, amylases, lipases, cutinases, cellulases, endoglucanases, xyloglucanases, pectinases, pectin lyases, xanthanases, peroxidaes, haloperoxygenases, catalases, mannanases, or any mixture thereof.

7. The composition of claim 5 or 6 comprising one or more components selected among surfactants, builders, etc.

8. An isolated polynucleotide encoding the polypeptide of any of claims 1-3.

9. A nucleic acid construct or expression vector comprising the polynucleotide of claim 8 operably linked to one or more control sequences that direct the production of the polypeptide in an expression host.

10. A recombinant host cell comprising the polynucleotide of claim 8 operably linked to one or more control sequences that direct the production of the polypeptide.

11. A method of producing the polypeptide of any of claims 1-3, comprising:

    (a) cultivating a cell, which in its wild-type form produces the polypeptide, under conditions conducive for production of the polypeptide; and
    (b) recovering the polypeptide.

12. A method of producing a polypeptide having protease activity according to claims 1-3, comprising:

(a) cultivating the host cell of claim 10 under conditions conducive for production of the polypeptide; and
(b) recovering the polypeptide.

**13.** A method for improving the nutritional value of an animal feed, wherein at least one protease of any one of claims 1-3 is added to the feed.

**14.** An animal feed additive comprising

a. at least one protease of any one of claims 1-3; and
b. at least one fat-soluble vitamin, and/or
c. at least one water-soluble vitamin, and/or
d. at least one trace mineral.

**15.** The animal feed additive of claim 14, which further comprises amylase, phytase, xylanase, galactanase, alpha-galactosidase, protease, phospholipase, and/or beta-glucanase.

**16.** An animal feed having a crude protein content of 50 to 800 g/kg and comprising at least one protease of any one of claims 1-3.

**17.** A method for the treatment of proteins, comprising the step of adding at least one protease of any one of claims 1-3 to at least one protein or protein source.

**18.** The method of claim 17, wherein soybean is included among at least one protein source.

**19.** Use of at least one protease of any one of claims 1-3 in detergents.

**20.** An animal feed composition comprising the polypeptide of any of claims 1-3.

**21.** The animal feed composition of claim 20, which further comprises one or more amylases, phytases, xylanases, galactanases, alpha-galactosidases, proteases, phospholipases, beta-glucanases or any mixture thereof.

**Patentansprüche**

**1.** Isoliertes Polypeptid mit Proteaseaktivität, ausgewählt aus der Gruppe bestehend aus:

(a) einem Polypeptid mit mindestens 85% Sequenzidentität zum reifen Polypeptid von SEQ ID NO: 2, wobei das reife Polypeptid Aminosäuren 189 bis 374 von SEQ ID NO: 2 ist;
(b) einem Polypeptid, das durch ein Polynukleotid mit mindestens 85% Sequenzidentität zur das reife Polypeptid kodierenden Sequenz von SEQ ID NO: 1 kodiert ist, wobei das reife Polynukleotid Nukleotide 665 bis 1222 von SEQ ID NO: 1 ist; und
(c) einem Fragment des Polypeptids von (a) oder (b), das Proteaseaktivität aufweist, wobei das Polypeptid verbesserte Proteaseaktivität zwischen 37°C und 60°C verglichen zur Protease 10R (SEQ ID NO: 7) aufweist.

**2.** Polypeptid nach Anspruch 1, umfassend oder bestehend aus SEQ ID NO: 2 oder das/dem reife(n) Polypeptid von SEQ ID NO: 2.

**3.** Polypeptid nach einem beliebigen der Ansprüche 1-2, das eine Variante des reifen Polypeptids von SEQ ID NO: 2 umfassend eine Substitution, Deletion und/oder Insertion an einer oder mehreren Positionen ist.

**4.** Zusammensetzung, umfassend das Polypeptid gemäß einem beliebigen der Ansprüche 1-3.

**5.** Zusammensetzung nach Anspruch 4, die eine Detergenszusammensetzung ist, wie eine Zusammensetzung für Wäschewaschen oder automatisiertes Geschirrspülen.

**6.** Zusammensetzung nach Anspruch 5, weiterhin umfassend ein oder mehrere zusätzliche Enzyme ausgewählt aus Proteasen, Amylasen, Lipasen, Cutinasen, Cellulasen, Endoglucanasen, Xyloglucanasen, Pektinasen, Pektinlyasen, Xanthanasen, Peroxidasen, Haloperoxygenasen, Katalasen, Mannanasen, oder einem beliebigen Gemisch

davon.

**7.** Zusammensetzung nach Anspruch 5 oder 6, umfassend einen oder mehrere Bestandteile ausgewählt aus oberflächenaktiven Mitteln, Gerüststoffen, etc.

**8.** Isoliertes Polynukleotid, das das Polypeptid gemäß einem beliebigen der Ansprüche 1-3 kodiert.

**9.** Nukleinsäurekonstrukt oder Expressionvektor, umfassend das Polynukleotid gemäß Anspruch 8, funktionsfähig verknüpft mit einer oder mehreren Kontrollsequenzen, die die Herstellung des Polypeptids in einem Expressionswirt steuert/steuern.

**10.** Rekombinante Wirtszelle, umfassend das Polynukleotid gemäß Anspruch 8 funktionsfähig verknüpft mit einer oder mehreren Kontrollsequenzen, die die Herstellung des Polypeptids steuert/steuern.

**11.** Verfahren zum Herstellen des Polypeptids gemäß einem beliebigen der Ansprüche 1-3, umfassend:

(a) Kultivieren einer Zelle, die in ihrer Wildtyp-Form das Polypeptid herstellt, unter Bedingungen, die für die Herstellung des Polypeptids förderlich sind; und
(b) Gewinnen des Polypeptids.

**12.** Verfahren zum Herstellen eines Polypeptids mit Proteaseaktivität gemäß Anspruch 1-3, umfassend:

(a) Kultivieren der Wirtszelle gemäß Anspruch 10 unter Bedingungen, die für die Herstellung des Polypeptids förderlich sind; und
(b) Gewinnen des Polypeptids.

**13.** Verfahren zum Verbessern des Nährwerts eines Tierfutters, wobei die mindestens eine Protease gemäß einem beliebigen der Ansprüche 1-3 zum Futter zugegeben wird.

**14.** Tierfutterzusatz, umfassend

a. mindestens eine Protease gemäß einem beliebigen der Ansprüche 1-3; und
b. mindestens ein fettlösliches Vitamin, und/oder
c. mindestens ein wasserlösliches Vitamin, und/oder
d. mindestens ein Spurenmineral.

**15.** Tierfutterzusatz nach Anspruch 14, der weiterhin Amylase, Phytase, Xylanase, Galactanase, alpha-Galactosidase, Protease, Phospholipase, und/oder beta-Glucanase umfasst.

**16.** Tierfutter mit einem Rohproteingehalt von 50 bis 800 g/kg und umfassend mindestens eine Protease gemäß einem beliebigen der Ansprüche 1-3.

**17.** Verfahren zur Behandlung von Proteinen, umfassend den Schritt des Zugebens von mindestens einer Protease gemäß einem beliebigen der Ansprüche 1-3 zu mindestens einem Protein oder einer Proteinquelle.

**18.** Verfahren nach Anspruch 17, wobei Sojabohne unter der mindestens einen Proteinquelle eingeschlossen ist.

**19.** Verwendung von mindestens einer Protease gemäß einem beliebigen der Ansprüche 1-3 in Detergentien.

**20.** Tierfutterzusammensetzung, umfassend das Polypeptid gemäß einem beliebigen der Ansprüche 1-3.

**21.** Tierfutterzusammensetzung nach Anspruch 20, die weiterhin eine oder mehrere Amylasen, Phytasen, Xylanasen, Galactanasen, alpha-Galactosidasen, Proteasen, Phospholipasen, beta-Glucanasen, oder ein beliebiges Gemisch davon umfasst.

## Revendications

1. Polypeptide isolé ayant une activité protéase, sélectionné dans le groupe consistant en :

   (a) un polypeptide présentant au moins 85 % d'identité de séquence avec le polypeptide mature de SEQ ID NO : 2, dans lequel le polypeptide mature correspond aux acides aminés 189 à 374 de SEQ ID NO : 2 ;
   (b) un polypeptide codé par un polynucléotide présentant au moins 85 % d'identité de séquence avec la séquence de SEQ ID NO : 1 codant pour le polypeptide mature, dans lequel le polynucléotide mature correspond aux nucléotides 665 à 1222 de SEQ ID NO : 1 ; et
   (c) un fragment du polypeptide de (a) ou (b) qui a une activité protéase,

   dans lequel le polypeptide a une activité protéase améliorée entre 37 °C et 60 °C, par rapport à la protéase 10R (SEQ ID NO : 7).

2. Polypeptide selon la revendication 1, comprenant ou consistant en SEQ ID NO : 2 ou le polypeptide mature de SEQ ID NO : 2.

3. Polypeptide selon l'une quelconque des revendications 1 à 2, qui est un variant du polypeptide mature de SEQ ID NO : 2 comprenant une substitution, une délétion, et/ou une insertion à une ou plusieurs positions.

4. Composition comprenant le polypeptide selon l'une quelconque des revendications 1 à 3.

5. Composition selon la revendication 4 qui est une composition détergente telle qu'une composition pour un lavage de linge ou un lavage de vaisselle automatisé.

6. Composition selon la revendication 5 comprenant en outre une ou plusieurs enzymes additionnelles sélectionnées parmi des protéases, des amylases, des lipases, des cutinases, des cellulases, des endoglucanases, des xyloglucanases, des pectinases, des pectine lyases, des xanthanases, des peroxydases, des halogénoperoxygénases, des catalases, mannanases, ou un mélange quelconque de celles-ci.

7. Composition selon la revendication 5 ou 6 comprenant un ou plusieurs composants sélectionnés parmi des tensioactifs, des adjuvants, etc.

8. Polynucléotide isolé codant pour le polypeptide selon l'une quelconque des revendications 1 à 3.

9. Construction d'acide nucléique ou vecteur d'expression comprenant le polynucléotide selon la revendication 8 lié de façon fonctionnelle à une ou plusieurs séquences de contrôle qui dirigent la production du polypeptide dans un hôte d'expression.

10. Cellule hôte recombinante comprenant le polynucléotide selon la revendication 8 lié de façon fonctionnelle à une ou plusieurs séquences de contrôle qui dirigent la production du polypeptide.

11. Méthode de production du polypeptide selon l'une quelconque des revendications 1 à 3, comprenant :

    (a) la culture d'une cellule, qui sous sa forme de type sauvage produit le polypeptide, dans des conditions propices à une production du polypeptide ; et
    (b) la récupération du polypeptide.

12. Méthode de production d'un polypeptide ayant une activité protéase selon les revendications 1 à 3, comprenant :

    (a) la culture de la cellule hôte selon la revendication 10 dans des conditions propices à une production du polypeptide ; et
    (b) la récupération du polypeptide.

13. Méthode d'amélioration de la valeur nutritionnelle d'un aliment pour animaux, dans laquelle au moins une protéase selon l'une quelconque des revendications 1 à 3 est ajoutée à l'aliment.

14. Additif d'aliment pour animaux comprenant

a. au moins une protéase selon l'une quelconque des revendications 1 à 3 ; et
b. au moins une vitamine liposoluble, et/ou
c. au moins une vitamine hydrosoluble, et/ou
d. au moins un oligo-élément.

**15.** Additif d'aliment pour animaux selon la revendication 14, qui comprend en outre une amylase, une phytase, une xylanase, une galactanase, une alpha-galactosidase, une protéase, une phospholipase, et/ou une bêta-glucanase.

**16.** Aliment pour animaux ayant une teneur en protéines brutes de 50 à 800 g/kg et comprenant au moins une protéase selon l'une quelconque des revendications 1 à 3.

**17.** Méthode pour le traitement des protéines, comprenant l'étape d'addition d'au moins une protéase selon l'une quelconque des revendications 1 à 3 à au moins une protéine ou une source de protéines.

**18.** Méthode selon la revendication 17, dans laquelle du soja est inclus parmi au moins une source de protéines.

**19.** Utilisation d'au moins une protéase selon l'une quelconque des revendications 1 à 3 dans des détergents.

**20.** Composition d'aliment pour animaux comprenant le polypeptide selon l'une quelconque des revendications 1 à 3.

**21.** Composition d'aliment pour animaux selon la revendication 20, qui comprend en outre une ou plusieurs amylases, phytases, xylanases, galactanases, alpha-galactosidases, protéases, phospholipases, bêta-glucanases ou un mélange quelconque de celles-ci.

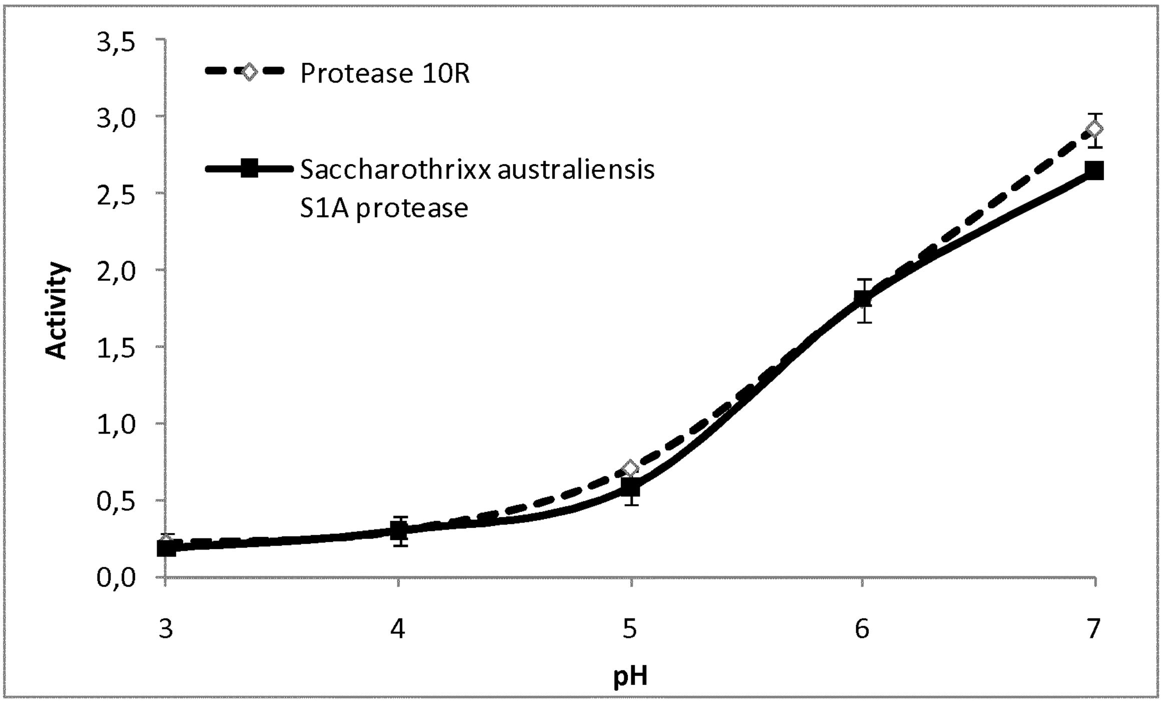
3,5
3,0
2,5
2,0
1,5
1,0
0,5
0,0
Activity
Protease 10R
Saccharothrixx australiensis
S1A protease
3
4
5
6
7
pH

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9528850 A
- WO 0158275 A
- WO 0158276 A
- WO 04072221 A
- WO 04111220 A
- WO 04111223 A
- WO 05035747 A
- WO 05123911 A
- WO 04072279 A
- WO 04034776 A
- WO 04077960 A
- WO 9517413 A
- WO 9522625 A
- US 5223409 A
- WO 9206204 A
- WO 9943835 A
- WO 9600787 A
- WO 0056900 A
- WO 9425612 A
- WO 9533836 A
- WO 0024883 A
- EP 238023 A
- WO 9114772 A
- US 6395966 B
- US 7151204 B
- WO 9219709 A
- WO 9219708 A
- WO 2005105826 A
- WO 2009118375 A
- WO 9707202 A
- WO 09102854 A
- US 5977053 A
- WO 9817767 A
- EP 624154 A
- WO 2007087258 A
- WO 2007087244 A
- WO 2007087259 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 00060063 A
- WO 2007087508 A
- WO 2009109500 A
- GB 1296839 A
- WO 9402597 A

- WO 9418314 A
- WO 9623873 A
- WO 9743424 A
- WO 9324618 A
- WO 9510602 A
- WO 9815257 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- US 20090011970 A1
- WO 09092699 A
- EP 1705241 A
- EP 1382668 A
- WO 07001262 A
- US 6472364 B
- WO 04074419 A
- WO 09124162 A
- WO 09124163 A
- WO 09117340 A
- WO 09117341 A
- WO 09117342 A
- WO 09072069 A
- WO 09063355 A
- WO 09132870 A
- WO 09121757 A
- WO 09112296 A
- WO 09112298 A
- WO 09103822 A
- WO 09087033 A
- WO 09050026 A
- WO 09047125 A
- WO 09047126 A
- WO 09047127 A
- WO 09047128 A
- WO 09021784 A
- WO 09010375 A
- WO 09000605 A
- WO 09122125 A
- WO 09095645 A
- WO 09040544 A
- WO 09040545 A
- WO 09024780 A
- WO 09004295 A
- WO 09004294 A
- WO 09121725 A
- WO 09115391 A
- WO 09115392 A
- WO 09074398 A
- WO 09074403 A
- WO 09068501 A
- WO 09065770 A
- WO 09021813 A
- WO 09030632 A
- WO 09015951 A
- WO 2011025615 A
- WO 2011016958 A
- WO 2011005803 A
- WO 2011005623 A
- WO 2011005730 A
- WO 2011005844 A
- WO 2011005904 A
- WO 2011005630 A
- WO 2011005830 A
- WO 2011005912 A
- WO 2011005905 A
- WO 2011005910 A
- WO 2011005813 A
- WO 2010135238 A
- WO 2010120863 A
- WO 2010108002 A
- WO 2010111365 A
- WO 2010108000 A
- WO 2010107635 A
- WO 2010090915 A
- WO 2010033976 A
- WO 2010033746 A
- WO 2010033747 A
- WO 2010033897 A
- WO 2010033979 A
- WO 2010030540 A
- WO 2010030541 A
- WO 2010030539 A
- WO 2010024467 A
- WO 2010024469 A
- WO 2010024470 A
- WO 2010025161 A
- WO 2010014395 A
- WO 2010044905 A
- WO 2010145887 A
- WO 2010142503 A
- WO 2010122051 A
- WO 2010102861 A
- WO 2010099997 A
- WO 2010084039 A
- WO 2010076292 A
- WO 2010069742 A
- WO 2010069718 A
- WO 2010069957 A
- WO 2010057784 A
- WO 2010054986 A
- WO 2010018043 A
- WO 2010003783 A
- WO 2010003792 A
- WO 2011023716 A
- WO 2010142539 A
- WO 2010118959 A
- WO 2010115813 A
- WO 2010105942 A
- WO 2010105961 A

- WO 2010105962 A
- WO 2010094356 A
- WO 2010084203 A
- WO 2010078979 A
- WO 2010072456 A
- WO 2010069905 A
- WO 2010076165 A
- WO 2010072603 A
- WO 2010066486 A
- WO 2010066631 A
- WO 2010066632 A
- WO 2010063689 A
- WO 2010060821 A
- WO 2010049187 A
- WO 2010031607 A
- WO 2010000636 A
- US 6077316 A
- WO 03044049 A
- WO 03048148 A
- WO 9401459 A
- WO 02090384 A
- US 09779334 B
- WO 0242740 A
- EP 2011064585 W
- WO 8803947 A


**Non-patent literature cited in the description**


- The EC number refers to Enzyme Nomenclature. Academic Press, 1992, vol. 1-5
- *Eur. J. Bio-chem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- *Biochem.J.,* 1993, vol. 290, 205-218
- **RAWLINGS, N.D. ; BARRETT, A.J. ; BATEMAN, A.** MEROPS: the peptidase database. *Nucleic Acids Res,* 2010, vol. 38, D227-D233
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet,* 2000, vol. 16, 276-277
- **RICE et al.** *EMBOSS: The European Molecular Biology Open Software Suite,* 2000
- **H. NEURATH ; R.L. HILL.** The Proteins,. Academic Press, 1979
- **CUNNINGHAM ; WELLS.** *Science,* 1989, vol. 244, 1081-1085
- **HILTON et al.** *J. Biol. Chem,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **INNIS et al.** PCR: A Guide to Methods and Application. Academic Press, 1990
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** Useful proteins from recombinant bacteria. *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990

- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun,* 1981, vol. 32, 1295-1297
- **CATT ; JOLLICK.** *Microbios,* 1991, vol. 68, 189-207
- **BUCKLEY et al.** *Appl. Environ. Microbiol,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. CAB International, 1995
- Biology and Activities of Yeast. Soc. App. Bacteriol. Symposium Series No. 9, 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Guide to Yeast Genetics and Molecular Biology. **BECKER ; GUARENTE.** Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **TAGUE et al.** *Plant Physiology,* 1988, vol. 86, 506
- **FRANCK et al.** *Cell,* 1980, vol. 21, 285-294
- **CHRISTENSEN et al.** *Plant Mol. Biol,* 1992, vol. 18, 675-689
- **ZHANG et al.** *Plant Cell,* 1991, vol. 3, 1155-1165
- **EDWARDS ; CORUZZI.** *Ann. Rev. Genet,* 1990, vol. 24, 275-303
- **ITO et al.** *Plant Mol. Biol,* 1994, vol. 24, 863-878
- **WU et al.** *Plant Cell Physiol.,* 1998, vol. 39, 885-889
- **CONRAD et al.** *J. Plant Physiol.,* 1998, vol. 152, 708-711
- **CHEN et al.** *Plant Cell Physiol.,* 1998, vol. 39, 935-941
- **KYOZUKA et al.** *Plant Physiol,* 1993, vol. 102, 991-1000
- **MITRA ; HIGGINS.** *Plant Mol. Biol.,* 1994, vol. 26, 85-93
- **KAGAYA et al.** *Mol. Gen. Genet,* 1995, vol. 248, 668-674
- **XU et al.** *Plant Mol. Biol,* 1993, vol. 22, 573-588
- **GASSER et al.** *Science,* 1990, vol. 244, 1293
- **POTRYKUS.** *Bio/Technology,* 1990, vol. 8, 535
- **SHIMAMOTO et al.** *Nature,* 1989, vol. 338, 274
- **HOOYKAS ; SCHILPEROORT.** *Plant Mol. Biol,* 1992, vol. 19, 15-38
- **CHRISTOU.** *Plant J.,* 1992, vol. 2, 275-281
- **SHIMAMOTO.** *Curr. Opin. Biotechnol.,* 1994, vol. 5, 158-162
- **VASIL et al.** *Bio/Technology,* 1992, vol. 10, 667-674
- **OMIRULLEH et al.** *Plant Mol. Biol,* 1993, vol. 21, 415-428
- **HODGDON ; KALER.** *Current Opinion in Colloid & Interface Science,* 2007, vol. 12, 121-128
- **DARTOIS et al.** *Biochemica et Biophysica Acta,* 1993, vol. 1131, 253-360
- Powdered Detergents, Surfactant science series. Marcel Dekker, Inc, vol. 71
- Official Methods of Analysis. Association of Official Analytical Chemists, 1984
- swine nutrition, committee on animal nutrition, board of agriculture, national research council. National Academy Press, 1988, 2-6
- **GRAFISCH BEDRIJF PONSEN ; LOOIJEN BV.** European Table of Energy Values for Poultry Feed-stuffs. Spelderholt centre for poultry research and extension
- **VEEVOEDERTABEL.** gegevens over chemische samenstelling, verteerbaarheid en voederwaarde van voedermiddelen. Central Veevoederbureau, 1997

- **NIELSEN, PM ; PETERSEN, D ; DAMPMANN, C.** Improved method for determining food protein degree of hydrolysis. *J Food Sci,* 2001, vol. 66, 642-646
- JMP® 5 Administrators Guide to Annually Licensed Windows, Mackintosh, and Linux Versions, Release 5.1. SAS Institute, 2003

SEQUENCE LISTING

<110> Novozymes A/S

<120> POLYPEPTIDES HAVING PROTEASE ACTIVITY AND POLYNUCLEOTIDES ENCODING SAME

<130> 12132-WO-PCT

<160> 7

<170> PatentIn version 3.5

<210> 1
<211> 1505
<212> DNA
<213> Saccharothrix australiensis

<220>
<221> CDS
<222> (101)..(1405)

<220>
<221> sig_peptide
<222> (101)..(187)

<400> 1

```
agttatgact cgactggcat atccgccacc tcgctcagcc ggctagcgtc gtccgcacgg       60

ccccgcatcc cgtgtcacca cccctgcaag gagacaacgg atg act cga cgc atc        115
                                            Met Thr Arg Arg Ile
                                            1               5

gcc gcc gcc gtc gcc gtg gcg gtc atg agc gct gcc ggt gtg gca gcc        163
Ala Ala Ala Val Ala Val Ala Val Met Ser Ala Ala Gly Val Ala Ala
                10                  15                  20

gct ctg acc acc gcc gcg aac gcc ggt cca ccg aca acg cac cag gag        211
Ala Leu Thr Thr Ala Ala Asn Ala Gly Pro Pro Thr Thr His Gln Glu
            25                  30                  35

gag agc ggc ctc atc gcc gcg atg gcg cgc gac ttc aag atc acg ccc        259
Glu Ser Gly Leu Ile Ala Ala Met Ala Arg Asp Phe Lys Ile Thr Pro
        40                  45                  50

gac cag gcg cgc gcc cgg ctc gtc cgg gag gcc aag gcc gcg acc acc        307
Asp Gln Ala Arg Ala Arg Leu Val Arg Glu Ala Lys Ala Ala Thr Thr
    55                  60                  65

gag cag agc ctg aag tcg cgg ctc ggc ggc cac tac gcg ggc gcc tgg        355
Glu Gln Ser Leu Lys Ser Arg Leu Gly Gly His Tyr Ala Gly Ala Trp
70                  75                  80                  85

ctg aac gag ggc gcc acc gaa ctc gtc gtc gcc gtc acc gac gcg gcg        403
Leu Asn Glu Gly Ala Thr Glu Leu Val Val Ala Val Thr Asp Ala Ala
                90                  95                  100

cag gcc aag gtg gtc gag gac gcc ggc gcg acg ccg aag gtc gtg cag        451
Gln Ala Lys Val Val Glu Asp Ala Gly Ala Thr Pro Lys Val Val Gln
            105                 110                 115

cgc agc cag atc cag ctc gac gag ctg aag gcc aag ctg gac gcg aac        499
```

```
Arg Ser Gln Ile Gln Leu Asp Glu Leu Lys Ala Lys Leu Asp Ala Asn
        120                 125                 130

aag aac gcg ccg aag gac gtg ccc gcg tgg tac gtc gac gtg aag acc      547
Lys Asn Ala Pro Lys Asp Val Pro Ala Trp Tyr Val Asp Val Lys Thr
    135                 140                 145

aac tcc gtg gtc gtg ctg gcc cgc aac acc gcg agc gcc aag gcg ttc      595
Asn Ser Val Val Val Leu Ala Arg Asn Thr Ala Ser Ala Lys Ala Phe
150                 155                 160                 165

gcc cgc gcc agc ggt ctg agc gag gcg gac gtc cgg atc gag cag tcc      643
Ala Arg Ala Ser Gly Leu Ser Glu Ala Asp Val Arg Ile Glu Gln Ser
                170                 175                 180

acc gag gac ccg cgc ccg ctg atc gac gtc atc ggc ggc aac gcg tac      691
Thr Glu Asp Pro Arg Pro Leu Ile Asp Val Ile Gly Gly Asn Ala Tyr
            185                 190                 195

tac atg ggc agc ggc ggt cgc tgc tcg gtc ggc ttc tcg gtc aac ggc      739
Tyr Met Gly Ser Gly Gly Arg Cys Ser Val Gly Phe Ser Val Asn Gly
        200                 205                 210

ggc ttc gtg acg gcg ggc cac tgc ggc cgg gtc ggc acc acg acc acc      787
Gly Phe Val Thr Ala Gly His Cys Gly Arg Val Gly Thr Thr Thr Thr
    215                 220                 225

cag ccc agc ggc acg ttc gcc ggt tcc acc ttc ccc ggc cgg gac tac      835
Gln Pro Ser Gly Thr Phe Ala Gly Ser Thr Phe Pro Gly Arg Asp Tyr
230                 235                 240                 245

gcc tgg gtc cgc gtg agc tcg ggc aac acc atg cgc ggc ctg gtc aac      883
Ala Trp Val Arg Val Ser Ser Gly Asn Thr Met Arg Gly Leu Val Asn
                250                 255                 260

cgc tac ccc ggc acc gtg ccg gtg aag ggc tcc aac gag tcg tcc gtc      931
Arg Tyr Pro Gly Thr Val Pro Val Lys Gly Ser Asn Glu Ser Ser Val
            265                 270                 275

ggc gcg tcg gtc tgc cgt tcc ggc tcg acg acg ggt tgg cac tgc ggc      979
Gly Ala Ser Val Cys Arg Ser Gly Ser Thr Thr Gly Trp His Cys Gly
        280                 285                 290

acc atc cag cag aag aac acc tcc gtg acg tac ccc gag ggc acc atc     1027
Thr Ile Gln Gln Lys Asn Thr Ser Val Thr Tyr Pro Glu Gly Thr Ile
    295                 300                 305

tcc ggt gtg acc cgg acc aac gcc tgc gcc gag ccc ggc gac tcg ggc     1075
Ser Gly Val Thr Arg Thr Asn Ala Cys Ala Glu Pro Gly Asp Ser Gly
310                 315                 320                 325

ggt tcg tgg ctc acc ggc gac cag gcc cag ggc gtc acc tcc ggt ggt     1123
Gly Ser Trp Leu Thr Gly Asp Gln Ala Gln Gly Val Thr Ser Gly Gly
                330                 335                 340

tcc ggg aac tgc tca tcc ggc ggc acg acg tac ttc cag ccg gtg aac     1171
Ser Gly Asn Cys Ser Ser Gly Gly Thr Thr Tyr Phe Gln Pro Val Asn
            345                 350                 355

ccg atc ctc cag gcg tac ggc ctc cag ctg gtc atc gag ggc ggc ccg     1219
Pro Ile Leu Gln Ala Tyr Gly Leu Gln Leu Val Ile Glu Gly Gly Pro
        360                 365                 370
```

```
acc ggc acc acc gga ccg acc acg acg tcc agc aac ccg ggc ggc acg       1267
Thr Gly Thr Thr Gly Pro Thr Thr Thr Ser Ser Asn Pro Gly Gly Thr
    375                 380                 385

acc tgg cag cca ggc gtc gcg tac acg gcc ggt acc acc gtc acg tac       1315
Thr Trp Gln Pro Gly Val Ala Tyr Thr Ala Gly Thr Thr Val Thr Tyr
390                 395                 400                 405

gag ggc gtc ggg tac gag tgc ttg cag ggg cac acg tcc caa atc ggc       1363
Glu Gly Val Gly Tyr Glu Cys Leu Gln Gly His Thr Ser Gln Ile Gly
                410                 415                 420

tgg gag ccg tcc gcg gtg ccc gct ctg tgg gag cgc gtg ggc               1405
Trp Glu Pro Ser Ala Val Pro Ala Leu Trp Glu Arg Val Gly
            425                 430                 435

tagcggcaag cttcgcgcgc gggggcgggg cccgactcgc gtcgggcccc ccttccccac     1465

gcgcctaccg ggatgacgg ggcgtttgcg gaggcgcccc                            1505
```

<210> 2
<211> 435
<212> PRT
<213> Saccharothrix australiensis

<400> 2

```
Met Thr Arg Arg Ile Ala Ala Ala Val Ala Val Ala Val Met Ser Ala
1               5                   10                  15

Ala Gly Val Ala Ala Ala Leu Thr Thr Ala Ala Asn Ala Gly Pro Pro
            20                  25                  30

Thr Thr His Gln Glu Glu Ser Gly Leu Ile Ala Ala Met Ala Arg Asp
        35                  40                  45

Phe Lys Ile Thr Pro Asp Gln Ala Arg Ala Arg Leu Val Arg Glu Ala
    50                  55                  60

Lys Ala Ala Thr Thr Glu Gln Ser Leu Lys Ser Arg Leu Gly Gly His
65                  70                  75                  80

Tyr Ala Gly Ala Trp Leu Asn Glu Gly Ala Thr Glu Leu Val Val Ala
                85                  90                  95

Val Thr Asp Ala Ala Gln Ala Lys Val Val Glu Asp Ala Gly Ala Thr
            100                 105                 110

Pro Lys Val Val Gln Arg Ser Gln Ile Gln Leu Asp Glu Leu Lys Ala
        115                 120                 125

Lys Leu Asp Ala Asn Lys Asn Ala Pro Lys Asp Val Pro Ala Trp Tyr
    130                 135                 140
```

Val Asp Val Lys Thr Asn Ser Val Val Val Leu Ala Arg Asn Thr Ala
145 150 155 160

Ser Ala Lys Ala Phe Ala Arg Ala Ser Gly Leu Ser Glu Ala Asp Val
165 170 175

Arg Ile Glu Gln Ser Thr Glu Asp Pro Arg Pro Leu Ile Asp Val Ile
180 185 190

Gly Gly Asn Ala Tyr Tyr Met Gly Ser Gly Gly Arg Cys Ser Val Gly
195 200 205

Phe Ser Val Asn Gly Gly Phe Val Thr Ala Gly His Cys Gly Arg Val
210 215 220

Gly Thr Thr Thr Thr Gln Pro Ser Gly Thr Phe Ala Gly Ser Thr Phe
225 230 235 240

Pro Gly Arg Asp Tyr Ala Trp Val Arg Val Ser Ser Gly Asn Thr Met
245 250 255

Arg Gly Leu Val Asn Arg Tyr Pro Gly Thr Val Pro Val Lys Gly Ser
260 265 270

Asn Glu Ser Ser Val Gly Ala Ser Val Cys Arg Ser Gly Ser Thr Thr
275 280 285

Gly Trp His Cys Gly Thr Ile Gln Gln Lys Asn Thr Ser Val Thr Tyr
290 295 300

Pro Glu Gly Thr Ile Ser Gly Val Thr Arg Thr Asn Ala Cys Ala Glu
305 310 315 320

Pro Gly Asp Ser Gly Gly Ser Trp Leu Thr Gly Asp Gln Ala Gln Gly
325 330 335

Val Thr Ser Gly Gly Ser Gly Asn Cys Ser Ser Gly Gly Thr Thr Tyr
340 345 350

Phe Gln Pro Val Asn Pro Ile Leu Gln Ala Tyr Gly Leu Gln Leu Val
355 360 365

Ile Glu Gly Gly Pro Thr Gly Thr Thr Gly Pro Thr Thr Thr Ser Ser
370 375 380

Asn Pro Gly Gly Thr Thr Trp Gln Pro Gly Val Ala Tyr Thr Ala Gly
385 390 395 400

```
Thr Thr Val Thr Tyr Glu Gly Val Gly Tyr Glu Cys Leu Gln Gly His
                405                 410                 415

Thr Ser Gln Ile Gly Trp Glu Pro Ser Ala Val Pro Ala Leu Trp Glu
            420                 425                 430

Arg Val Gly
        435
```

<210> 3
<211> 1305
<212> DNA
<213> Artificial

<220>
<223> Synthetic gene

<400> 3

```
atgacacgtc gcattgccgc tgcagttgca gtagctgtaa tgtctgcggc aggagttgct      60
gcagctctta cgacggctgc taacgctgga cctccaacta cgcatcagga agaatcagga     120
cttatcgctg caatggctcg tgactttaag attacaccgg atcaagcacg tgctcgcctt     180
gtacgtgaag cgaaagcagc aactacagaa caatctttga aatctcgttt gggaggccac     240
tatgctggcg catggcttaa cgaaggtgct actgaattag tagttgctgt tactgatgcc     300
gcacaagcga aagttgttga agatgcggga gctacgccta aggtagtaca acgttcacaa     360
atccaattgg acgagcttaa agcaaaactt gacgctaaca aaaacgctcc aaaagacgta     420
cctgcatggt atgttgatgt aaagacaaat tcagtagtag ttcttgctcg taacacagcg     480
tctgcaaaag catttgctcg cgcatctgga ttatcagaag ctgacgttcg tatcgaacag     540
tctactgaag acccacgtcc gttaatcgac gttattggtg gcaatgcgta ttacatgggt     600
tctggaggcc gttgctcagt aggattttct gtaaatggcg gtttcgttac agctggtcac     660
tgtggtcgtg taggcacaac tactacacaa ccttctggta cttttgctgg ctctactttc     720
ccaggtcgcg actatgcttg ggttcgtgtt tcttctggaa atacgatgcg tggacttgta     780
aatcgttacc ctggtactgt acctgttaaa ggatctaatg aatcatcagt aggcgcttct     840
gtttgtcgtt ctggcagcac gacaggttgg cactgcggaa ctattcagca gaaaaacaca     900
tctgttactt accctgaggg aactatttct ggagtaactc gtacaaatgc gtgcgcagaa     960
cctggtgact ctggtggatc atggcttact ggtgatcagg ctcaaggagt aacatctgga    1020
ggctctggta actgctcttc tggtggcaca acgtatttcc aacctgttaa cccaatctta    1080
caagcatacg gccttcaatt agttattgaa ggtggaccaa ctggtacaac aggtcctacg    1140
acaacatctt ctaaccctgg aggcacaact tggcaacctg gtgttgcata cacggctggc    1200
```

actactgtta cgtacgaggg cgttggttac gaatgccttc aaggtcacac atctcagatc 1260

ggctgggaac ctagcgcagt tcctgccctt tgggaacgtg ttggc 1305

<210> 4
<211> 7
<212> PRT
<213> Saccharothrix australiensis

<400> 4

Ile Asp Val Ile Gly Gly Asn
1 5

<210> 5
<211> 186
<212> PRT
<213> Saccharothrix australiensis

<400> 5

```
Ile Asp Val Ile Gly Gly Asn Ala Tyr Tyr Met Gly Ser Gly Gly Arg
1               5                   10                  15

Cys Ser Val Gly Phe Ser Val Asn Gly Gly Phe Val Thr Ala Gly His
            20                  25                  30

Cys Gly Arg Val Gly Thr Thr Thr Thr Gln Pro Ser Gly Thr Phe Ala
        35                  40                  45

Gly Ser Thr Phe Pro Gly Arg Asp Tyr Ala Trp Val Arg Val Ser Ser
    50                  55                  60

Gly Asn Thr Met Arg Gly Leu Val Asn Arg Tyr Pro Gly Thr Val Pro
65                  70                  75                  80

Val Lys Gly Ser Asn Glu Ser Ser Val Gly Ala Ser Val Cys Arg Ser
                85                  90                  95

Gly Ser Thr Thr Gly Trp His Cys Gly Thr Ile Gln Gln Lys Asn Thr
            100                 105                 110

Ser Val Thr Tyr Pro Glu Gly Thr Ile Ser Gly Val Thr Arg Thr Asn
        115                 120                 125

Ala Cys Ala Glu Pro Gly Asp Ser Gly Gly Ser Trp Leu Thr Gly Asp
    130                 135                 140

Gln Ala Gln Gly Val Thr Ser Gly Gly Ser Gly Asn Cys Ser Ser Gly
145                 150                 155                 160

Gly Thr Thr Tyr Phe Gln Pro Val Asn Pro Ile Leu Gln Ala Tyr Gly
                165                 170                 175

Leu Gln Leu Val Ile Glu Gly Gly Pro Thr
            180                 185
```

<210> 6
<211> 269
<212> PRT
<213> Bacillus clausii

<400> 6

```
Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190
```

Val Ala Pro Gly Val Asn Val Gln Ser Thr Tyr Pro Gly Ser Thr Tyr
195 200 205

Ala Ser Leu Asn Gly Thr Ser Met Ala Thr Pro His Val Ala Gly Ala
210 215 220

Ala Ala Leu Val Lys Gln Lys Asn Pro Ser Trp Ser Asn Val Gln Ile
225 230 235 240

Arg Asn His Leu Lys Asn Thr Ala Thr Ser Leu Gly Ser Thr Asn Leu
245 250 255

Tyr Gly Ser Gly Leu Val Asn Ala Glu Ala Ala Thr Arg
260 265

<210> 7
<211> 188
<212> PRT
<213> Nocardiopsis sp. (protease 10)

<400> 7

Ala Asp Ile Ile Gly Gly Leu Ala Tyr Thr Met Gly Gly Arg Cys Ser
1 5 10 15

Val Gly Phe Ala Ala Thr Asn Ala Ala Gly Gln Pro Gly Phe Val Thr
20 25 30

Ala Gly His Cys Gly Arg Val Gly Thr Gln Val Thr Ile Gly Asn Gly
35 40 45

Arg Gly Val Phe Glu Gln Ser Val Phe Pro Gly Asn Asp Ala Ala Phe
50 55 60

Val Arg Gly Thr Ser Asn Phe Thr Leu Thr Asn Leu Val Ser Arg Tyr
65 70 75 80

Asn Thr Gly Gly Tyr Ala Thr Val Ala Gly His Asn Gln Ala Pro Ile
85 90 95

Gly Ser Ser Val Cys Arg Ser Gly Ser Thr Thr Gly Trp His Cys Gly
100 105 110

Thr Ile Gln Ala Arg Gly Gln Ser Val Ser Tyr Pro Glu Gly Thr Val
115 120 125

Thr Asn Met Thr Arg Thr Thr Val Cys Ala Glu Pro Gly Asp Ser Gly
130 135 140

```
Gly Ser Tyr Ile Ser Gly Thr Gln Ala Gln Gly Val Thr Ser Gly Gly
145                 150                 155                 160

Ser Gly Asn Cys Arg Thr Gly Gly Thr Thr Phe Tyr Gln Glu Val Thr
                165                 170                 175

Pro Met Val Asn Ser Trp Gly Val Arg Leu Arg Thr
            180                 185
```